# EUROPEAN PATENT APPLICATION

(11) **EP 1 624 069 A1**
(43) Date of publication of application: **08.02.2006**
(21) Application number: 04731716.9
(22) Date of filing: 07.05.2004
(51) Int. Cl.: C12P 13/14, C12N 15/09

(54) **PROCESS FOR PRODUCING L-GLUTAMIC ACID**

(30) Priority: 07.05.2003 JP 2003128722
(71) Applicant: Ajinomoto Co., Inc., Chuo-ku, Tokyo 104-8315 (JP)
(72) Inventor: UEDA, Hiroshi, c/o AJINOMOTO CO., INC., Kanagawa, 2108681 (JP); TOUMORI, Kunihiko, c/o AJINOMOTO CO., INC., Kawasaki-shi, Kanagawa 2108681 (JP)
(74) Representative: Strehl Schübel-Hopf & Partner
(86) International application number: PCT/JP2004/006031
(87) International publication number: WO 2004/099426

(57) **Abstract**

In a method for producing L-glutamic acid by fermentation, which comprises culturing a microorganism that can metabolize a carbon source at a specific pH in a liquid medium containing L-glutamic acid at a saturation concentration and the carbon source and has an ability to accumulate L-glutamic acid in a liquid medium having said pH in an amount exceeding the amount corresponding to said saturation concentration of L-glutamic acid in a medium of which pH is controlled so that L-glutamic acid is precipitated to cause accumulation of L-glutamic acid in the medium while precipitating the L-glutamic acid, L-lysine is made exist in the medium when L-glutaminc acid concentration in the medium is lower than the concentration at which natural crystallization of L-glutamic acid occurs so that a-form crystals of L-glutamic acid is precipitated.

SEQUENCE LISTING

<110> Ajinomoto Co., Inc.

<120> Method for producing L-glutamic acid

<130> C2330PC4010

<150> JP 2003-128722
<151> 2003-05-07

<160> 13

<170> PatentIn Ver. 2.0

<210> 1
<211> 4556
<212> DNA
<213> Pantoea ananatis

<220>
<221> CDS
<222> (2)..(121)

<220>
<221> CDS
<222> (322)..(3129)

<220>
<221> CDS
<222> (3145)..(4368)

<220>
<221> CDS
<222> (4437)..(4556)

<400> 1
t gca ttc agc gtt ttc cgc tgt cac agc atc atg aac tgt gta agt gtt 49
  Ala Phe Ser Val Phe Arg Cys His Ser Ile Met Asn Cys Val Ser Val
    1               5                  10                  15
tgt cct aaa ggg cta aac ccg acg cgc gct atc ggc cac att aag tcg   97
Cys Pro Lys Gly Leu Asn Pro Thr Arg Ala Ile Gly His Ile Lys Ser

20                  25                  30
atg ctg ctg caa cgc agc gcg tagttatacc accgggaacc tcaggttccc     148
Met Leu Leu Gln Arg Ser Ala
        35
ggtattttac ggaagcctct gtaaacgcgg tcccaaccac gtttacaaag gttcccttac 208
gggccgggcg cgcgctgcgc acagtgctcg tatcgctgaa ctcactacgg caaaccgcga 268
aagcggcaac aaatgaaacc tcaaaaaagc ataacattgc ttaagggatc aca atg    324
                                                           Met
                                                            1
cag aac agc gcg atg aag ccc tgg ctg gac tcc tcc tgg ctg gcc ggc   372
Gln Asn Ser Ala Met Lys Pro Trp Leu Asp Ser Ser Trp Leu Ala Gly
            5                   10                  15
gcg aat cag tct tac ata gag caa ctc tat gag gat ttc ctg acc gat   420
Ala Asn Gln Ser Tyr Ile Glu Gln Leu Tyr Glu Asp Phe Leu Thr Asp
        20                  25                  30
cct gac tct gtg gat gca gtg tgg cgc tcg atg ttc caa cag tta cca   468
Pro Asp Ser Val Asp Ala Val Trp Arg Ser Met Phe Gln Gln Leu Pro
    35                  40                  45
ggc acg gga gtg aaa cct gag cag ttc cac tcc gca act cgc gaa tat   516
Gly Thr Gly Val Lys Pro Glu Gln Phe His Ser Ala Thr Arg Glu Tyr
50                  55                  60                  65
ttc cgt cgc ctg gcg aaa gac gca tct cgt tac acc tcc tca gtt acc   564
Phe Arg Arg Leu Ala Lys Asp Ala Ser Arg Tyr Thr Ser Ser Val Thr
                70                  75                  80
gat ccg gca acc aac tcc aaa caa gtg aaa gtg ctg cag ctg att aac   612
Asp Pro Ala Thr Asn Ser Lys Gln Val Lys Val Leu Gln Leu Ile Asn
            85                  90                  95
gcg ttt cgt ttc cgc gga cat cag gaa gca aat ctc gat ccg ctt ggc   660
Ala Phe Arg Phe Arg Gly His Gln Glu Ala Asn Leu Asp Pro Leu Gly
        100                 105                 110
ctg tgg aaa cag gac cgc gtt gcc gat ctc gat cct gcc ttt cac gat   708
Leu Trp Lys Gln Asp Arg Val Ala Asp Leu Asp Pro Ala Phe His Asp
    115                 120                 125
ctg acc gac gcc gat ttt cag gaa agc ttt aac gta ggt tct ttt gcc   756
Leu Thr Asp Ala Asp Phe Gln Glu Ser Phe Asn Val Gly Ser Phe Ala
130                 135                 140                 145
att ggc aaa gaa acc atg aag ctg gcc gat ctg ttc gac gcg ctg aag   804
Ile Gly Lys Glu Thr Met Lys Leu Ala Asp Leu Phe Asp Ala Leu Lys
                150                 155                 160
cag acc tac tgt ggc tcg att ggt gca gag tat atg cac atc aat aac   852
Gln Thr Tyr Cys Gly Ser Ile Gly Ala Glu Tyr Met His Ile Asn Asn
            165                 170                 175

acc gaa gag aaa cgc tgg atc cag cag cgt atc gaa tcc ggt gcg agc 900
Thr Glu Glu Lys Arg Trp Ile Gln Gln Arg Ile Glu Ser Gly Ala Ser
180 185 190
cag acg tca ttc agt ggc gaa gag aaa aaa ggt ttc ctg aaa gag ctg 948
Gln Thr Ser Phe Ser Gly Glu Glu Lys Lys Gly Phe Leu Lys Glu Leu
195 200 205
acc gcg gca gaa ggg ctg gaa aaa tat ctg ggc gcg aaa ttc ccg ggt 996
Thr Ala Ala Glu Gly Leu Glu Lys Tyr Leu Gly Ala Lys Phe Pro Gly
210 215 220 225
gca aaa cgt ttc tcg ctg gaa ggc ggt gat gcg ctg gtg ccg atg ctg 1044
Ala Lys Arg Phe Ser Leu Glu Gly Gly Asp Ala Leu Val Pro Met Leu
230 235 240
cgc gag atg att cgt cat gcg ggc aaa agc ggc aca cgt gaa gtg gta 1092
Arg Glu Met Ile Arg His Ala Gly Lys Ser Gly Thr Arg Glu Val Val
245 250 255
ctg ggg atg gcg cac cgt ggc cgt ctt aac gta ctg att aac gta ctg 1140
Leu Gly Met Ala His Arg Gly Arg Leu Asn Val Leu Ile Asn Val Leu
260 265 270
ggt aaa aag cca cag gat ctg ttc gac gaa ttc tcc ggt aaa cac aaa 1188
Gly Lys Lys Pro Gln Asp Leu Phe Asp Glu Phe Ser Gly Lys His Lys
275 280 285
gag cat ctg ggc acc ggt gat gtg aag tat cac atg ggc ttc tct tcg 1236
Glu His Leu Gly Thr Gly Asp Val Lys Tyr His Met Gly Phe Ser Ser
290 295 300 305
gat att gaa acc gaa ggt ggt ctg gtg cat ctg gcg ctg gcg ttt aac 1284
Asp Ile Glu Thr Glu Gly Gly Leu Val His Leu Ala Leu Ala Phe Asn
310 315 320
ccg tct cac ctg gaa att gtc agc ccg gtg gtc atg gga tcg gta cgt 1332
Pro Ser His Leu Glu Ile Val Ser Pro Val Val Met Gly Ser Val Arg
325 330 335
gca cgt ctc gat cgt ctg gcc gaa ccg gtc agc aat aaa gtg ttg cct 1380
Ala Arg Leu Asp Arg Leu Ala Glu Pro Val Ser Asn Lys Val Leu Pro
340 345 350
atc acc att cac ggt gat gcg gcg gtg att ggt cag ggc gtg gtt cag 1428
Ile Thr Ile His Gly Asp Ala Ala Val Ile Gly Gln Gly Val Val Gln
355 360 365
gaa acc ctg aac atg tct cag gcg cgc ggc tac gaa gtg ggc ggc acg 1476
Glu Thr Leu Asn Met Ser Gln Ala Arg Gly Tyr Glu Val Gly Gly Thr
370 375 380 385
gta cgt atc gtc att aac aac cag gtt ggt ttt acc acc tcc aac ccg 1524
Val Arg Ile Val Ile Asn Asn Gln Val Gly Phe Thr Thr Ser Asn Pro
390 395 400

aaa gat gcg cgt tca acc ccg tac tgt act gac atc ggc aag atg gtg    1572
Lys Asp Ala Arg Ser Thr Pro Tyr Cys Thr Asp Ile Gly Lys Met Val
            405                 410                 415
ctg gca ccg att ttc cac gtc aat gct gac gat ccg gaa gcg gtg gcc    1620
Leu Ala Pro Ile Phe His Val Asn Ala Asp Asp Pro Glu Ala Val Ala
        420                 425                 430
ttt gtt acc cgc ctg gcg ctg gac tat cgc aac acc ttc aaa cgc gat    1668
Phe Val Thr Arg Leu Ala Leu Asp Tyr Arg Asn Thr Phe Lys Arg Asp
    435                 440                 445
gtg ttt atc gat ctg gtg tgc tat cgc cgt cat ggt cac aac gag gcg    1716
Val Phe Ile Asp Leu Val Cys Tyr Arg Arg His Gly His Asn Glu Ala
450                 455                 460                 465
gat gag cca agt gct acc cag ccg ttg atg tac cag aaa atc aaa aag    1764
Asp Glu Pro Ser Ala Thr Gln Pro Leu Met Tyr Gln Lys Ile Lys Lys
                470                 475                 480
cat ccg acg ccg cgt aaa att tac gcc gat cgt ctg gaa ggc gaa ggt    1812
His Pro Thr Pro Arg Lys Ile Tyr Ala Asp Arg Leu Glu Gly Glu Gly
            485                 490                 495
gtc gcg tcg cag gaa gat gcc acc gag atg gtg aac ctg tac cgc gat    1860
Val Ala Ser Gln Glu Asp Ala Thr Glu Met Val Asn Leu Tyr Arg Asp
        500                 505                 510
gcg ctc gat gcg ggc gaa tgc gtg gtg ccg gaa tgg cgt ccg atg agc    1908
Ala Leu Asp Ala Gly Glu Cys Val Val Pro Glu Trp Arg Pro Met Ser
    515                 520                 525
ctg cac tcc ttc acg tgg tcg cct tat ctg aac cac gaa tgg gat gag    1956
Leu His Ser Phe Thr Trp Ser Pro Tyr Leu Asn His Glu Trp Asp Glu
530                 535                 540                 545
cct tat ccg gca cag gtt gac atg aaa cgc ctg aag gaa ctg gca ttg    2004
Pro Tyr Pro Ala Gln Val Asp Met Lys Arg Leu Lys Glu Leu Ala Leu
                550                 555                 560
cgt atc agc cag gtc cct gag cag att gaa gtg cag tcg cgc gtg gcc    2052
Arg Ile Ser Gln Val Pro Glu Gln Ile Glu Val Gln Ser Arg Val Ala
            565                 570                 575
aag atc tat aac gat cgc aag ctg atg gcc gaa ggc gag aaa gcg ttc    2100
Lys Ile Tyr Asn Asp Arg Lys Leu Met Ala Glu Gly Glu Lys Ala Phe
        580                 585                 590
gac tgg ggc ggt gcc gag aat ctg gcg tac gcc acg ctg gtg gat gaa    2148
Asp Trp Gly Gly Ala Glu Asn Leu Ala Tyr Ala Thr Leu Val Asp Glu
    595                 600                 605
ggt att ccg gtt cgc ctc tcg ggt gaa gac tcc ggt cgt gga acc ttc    2196
Gly Ile Pro Val Arg Leu Ser Gly Glu Asp Ser Gly Arg Gly Thr Phe
610                 615                 620                 625

ttc cat cgc cac gcg gtc gtg cac aac cag gct aac ggt tca acc tat 2244
Phe His Arg His Ala Val Val His Asn Gln Ala Asn Gly Ser Thr Tyr
630 635 640
acg ccg ctg cac cat att cat aac agc cag ggc gag ttc aaa gtc tgg 2292
Thr Pro Leu His His Ile His Asn Ser Gln Gly Glu Phe Lys Val Trp
645 650 655
gat tcg gtg ctg tct gaa gaa gcg gtg ctg gcg ttt gaa tac ggt tac 2340
Asp Ser Val Leu Ser Glu Glu Ala Val Leu Ala Phe Glu Tyr Gly Tyr
660 665 670
gcc acg gct gag ccg cgc gtg ctg acc atc tgg gaa gcg cag ttt ggt 2388
Ala Thr Ala Glu Pro Arg Val Leu Thr Ile Trp Glu Ala Gln Phe Gly
675 680 685
gac ttt gcc aac ggt gct cag gtg gtg att gac cag ttc atc agc tct 2436
Asp Phe Ala Asn Gly Ala Gln Val Val Ile Asp Gln Phe Ile Ser Ser
690 695 700 705
ggc gaa cag aag tgg ggc cgt atg tgt ggc ctg gtg atg ttg ctg ccg 2484
Gly Glu Gln Lys Trp Gly Arg Met Cys Gly Leu Val Met Leu Leu Pro
710 715 720
cat ggc tac gaa ggt cag gga ccg gaa cac tcc tct gcc cgt ctg gaa 2532
His Gly Tyr Glu Gly Gln Gly Pro Glu His Ser Ser Ala Arg Leu Glu
725 730 735
cgc tat ctg caa ctt tgc gcc gag cag aac atg cag gtt tgc gtc ccg 2580
Arg Tyr Leu Gln Leu Cys Ala Glu Gln Asn Met Gln Val Cys Val Pro
740 745 750
tcg acg ccg gct cag gtg tat cac atg ctg cgc cgt cag gcg ctg cgc 2628
Ser Thr Pro Ala Gln Val Tyr His Met Leu Arg Arg Gln Ala Leu Arg
755 760 765
ggg atg cgc cgt ccg ctg gtg gtg atg tcg ccg aag tcg ctg tta cgc 2676
Gly Met Arg Arg Pro Leu Val Val Met Ser Pro Lys Ser Leu Leu Arg
770 775 780 785
cat cca ctg gcg atc tcg tcg ctg gat gaa ctg gca aac ggc agt ttc 2724
His Pro Leu Ala Ile Ser Ser Leu Asp Glu Leu Ala Asn Gly Ser Phe
790 795 800
cag ccg gcc att ggt gag atc gac gat ctg gat ccg cag ggc gtg aaa 2772
Gln Pro Ala Ile Gly Glu Ile Asp Asp Leu Asp Pro Gln Gly Val Lys
805 810 815
cgc gtc gtg ctg tgc tcc ggt aag gtt tac tac gat ctg ctg gaa cag 2820
Arg Val Val Leu Cys Ser Gly Lys Val Tyr Tyr Asp Leu Leu Glu Gln
820 825 830
cgt cgt aaa gac gag aaa acc gat gtt gcc atc gtg cgc atc gaa cag 2868
Arg Arg Lys Asp Glu Lys Thr Asp Val Ala Ile Val Arg Ile Glu Gln
835 840 845

ctt tac ccg ttc ccg cat cag gcg gta cag gaa gca ttg aaa gcc tat 2916
Leu Tyr Pro Phe Pro His Gln Ala Val Gln Glu Ala Leu Lys Ala Tyr
850 855 860 865
tct cac gta cag gac ttt gtc tgg tgc cag gaa gag cct ctg aac cag 2964
Ser His Val Gln Asp Phe Val Trp Cys Gln Glu Glu Pro Leu Asn Gln
870 875 880
ggc gcc tgg tac tgt agc cag cat cat ttc cgt gat gtc gtg ccg ttt 3012
Gly Ala Trp Tyr Cys Ser Gln His His Phe Arg Asp Val Val Pro Phe
885 890 895
ggt gcc acc ctg cgt tat gca ggt cgc ccg gca tcg gct tct ccg gcc 3060
Gly Ala Thr Leu Arg Tyr Ala Gly Arg Pro Ala Ser Ala Ser Pro Ala
900 905 910
gtg ggt tat atg tcc gta cac caa caa cag cag caa gac ctg gtt aat 3108
Val Gly Tyr Met Ser Val His Gln Gln Gln Gln Gln Asp Leu Val Asn
915 920 925
gac gca ctg aac gtc aat taattaaaag gaaagata atg agt agc gta gat 3159
Asp Ala Leu Asn Val Asn Met Ser Ser Val Asp
930 935 1 5
att ctc gtt ccc gac ctg cct gaa tcg gtt gca gat gcc aca gta gca 3207
Ile Leu Val Pro Asp Leu Pro Glu Ser Val Ala Asp Ala Thr Val Ala
10 15 20
acc tgg cac aag aaa cca ggc gat gca gtc agc cgc gat gaa gtc atc 3255
Thr Trp His Lys Lys Pro Gly Asp Ala Val Ser Arg Asp Glu Val Ile
25 30 35
gtc gaa att gaa act gac aaa gtc gtg ctg gaa gtg ccg gca tct gcc 3303
Val Glu Ile Glu Thr Asp Lys Val Val Leu Glu Val Pro Ala Ser Ala
40 45 50
gat ggc gtg ctg gaa gcc gtg ctg gaa gac gaa ggg gca acc gtt acg 3351
Asp Gly Val Leu Glu Ala Val Leu Glu Asp Glu Gly Ala Thr Val Thr
55 60 65
tcc cgc cag atc ctg ggt cgc ctg aaa gaa ggc aac agt gcg ggt aaa 3399
Ser Arg Gln Ile Leu Gly Arg Leu Lys Glu Gly Asn Ser Ala Gly Lys
70 75 80 85
gaa agc agt gcc aaa gcg gaa agc aat gac acc acg cca gcc cag cgt 3447
Glu Ser Ser Ala Lys Ala Glu Ser Asn Asp Thr Thr Pro Ala Gln Arg
90 95 100
cag aca gcg tcg ctt gaa gaa gag agc agc gat gcg ctc agc ccg gcg 3495
Gln Thr Ala Ser Leu Glu Glu Glu Ser Ser Asp Ala Leu Ser Pro Ala
105 110 115
atc cgt cgc ctg att gcg gag cat aat ctt gac gct gcg cag atc aaa 3543
Ile Arg Arg Leu Ile Ala Glu His Asn Leu Asp Ala Ala Gln Ile Lys
120 125 130

ggc acc ggc gta ggc gga cgt tta acg cgt gaa gac gtt gaa aaa cat 3591
Gly Thr Gly Val Gly Gly Arg Leu Thr Arg Glu Asp Val Glu Lys His
135 140 145
ctg gcg aac aaa ccg cag gct gag aaa gcc gcc gcg cca gcg gcg ggt 3639
Leu Ala Asn Lys Pro Gln Ala Glu Lys Ala Ala Ala Pro Ala Ala Gly
150 155 160 165
gca gca acg gct cag cag cct gtt gcc aac cgc agc gaa aaa cgt gtt 3687
Ala Ala Thr Ala Gln Gln Pro Val Ala Asn Arg Ser Glu Lys Arg Val
170 175 180
ccg atg acg cgt tta cgt aag cgc gtc gcg gag cgt ctg ctg gaa gcc 3735
Pro Met Thr Arg Leu Arg Lys Arg Val Ala Glu Arg Leu Leu Glu Ala
185 190 195
aag aac agc acc gcc atg ttg acg acc ttc aac gaa atc aac atg aag 3783
Lys Asn Ser Thr Ala Met Leu Thr Thr Phe Asn Glu Ile Asn Met Lys
200 205 210
ccg att atg gat ctg cgt aag cag tac ggc gat gcg ttc gag aag cgt 3831
Pro Ile Met Asp Leu Arg Lys Gln Tyr Gly Asp Ala Phe Glu Lys Arg
215 220 225
cac ggt gtg cgt ctg ggc ttt atg tct ttc tac atc aag gcc gtg gtc 3879
His Gly Val Arg Leu Gly Phe Met Ser Phe Tyr Ile Lys Ala Val Val
230 235 240 245
gaa gcg ctg aag cgt tat cca gaa gtc aac gcc tct atc gat ggc gaa 3927
Glu Ala Leu Lys Arg Tyr Pro Glu Val Asn Ala Ser Ile Asp Gly Glu
250 255 260
gac gtg gtg tac cac aac tat ttc gat gtg agt att gcc gtc tct acg 3975
Asp Val Val Tyr His Asn Tyr Phe Asp Val Ser Ile Ala Val Ser Thr
265 270 275
cca cgc gga ctg gtg acg cct gtc ctg cgt gac gtt gat gcg ctg agc 4023
Pro Arg Gly Leu Val Thr Pro Val Leu Arg Asp Val Asp Ala Leu Ser
280 285 290
atg gct gac atc gag aag aaa att aaa gaa ctg gca gtg aaa ggc cgt 4071
Met Ala Asp Ile Glu Lys Lys Ile Lys Glu Leu Ala Val Lys Gly Arg
295 300 305
gac ggc aag ctg acg gtt gac gat ctg acg ggc ggt aac ttt acc atc 4119
Asp Gly Lys Leu Thr Val Asp Asp Leu Thr Gly Gly Asn Phe Thr Ile
310 315 320 325
acc aac ggt ggt gtg ttc ggt tcg ctg atg tct acg cca atc atc aac 4167
Thr Asn Gly Gly Val Phe Gly Ser Leu Met Ser Thr Pro Ile Ile Asn
330 335 340
ccg cca cag agc gcg att ctg ggc atg cac gcc att aaa gat cgt cct 4215
Pro Pro Gln Ser Ala Ile Leu Gly Met His Ala Ile Lys Asp Arg Pro
345 350 355

atg gcg gtc aat ggt cag gtt gtg atc ctg cca atg atg tac ctg gct    4263
Met Ala Val Asn Gly Gln Val Val Ile Leu Pro Met Met Tyr Leu Ala
        360                 365                 370
ctc tcc tac gat cac cgt tta atc gat ggt cgt gaa tct gtc ggc tat    4311
Leu Ser Tyr Asp His Arg Leu Ile Asp Gly Arg Glu Ser Val Gly Tyr
    375                 380                 385
ctg gtc gcg gtg aaa gag atg ctg gaa gat ccg gcg cgt ctg ctg ctg    4359
Leu Val Ala Val Lys Glu Met Leu Glu Asp Pro Ala Arg Leu Leu Leu
390                 395                 400                 405
gat gtc tgattcatca ctgggcacgc gttgcgtgcc caatctcaat actcttttca    4415
Asp Val

gatctgaatg gatagaacat c atg aac tta cac gaa tac cag gct aaa cag    4466
                        Met Asn Leu His Glu Tyr Gln Ala Lys Gln
                        1               5                   10
ctg ttt gca cgg tat ggc atg cca gca ccg acc ggc tac gcc tgt act    4514
Leu Phe Ala Arg Tyr Gly Met Pro Ala Pro Thr Gly Tyr Ala Cys Thr
                15                  20                  25
aca cca cgt gaa gca gaa gaa gcg gca tcg aaa atc ggt gca            4556
Thr Pro Arg Glu Ala Glu Glu Ala Ala Ser Lys Ile Gly Ala
            30                  35                  40

<210> 2
<211> 39
<212> PRT
<213> Pantoea ananatis

<400> 2
Ala Phe Ser Val Phe Arg Cys His Ser Ile Met Asn Cys Val Ser Val
1               5                   10                  15
Cys Pro Lys Gly Leu Asn Pro Thr Arg Ala Ile Gly His Ile Lys Ser
            20                  25                  30
Met Leu Leu Gln Arg Ser Ala
        35

<210> 3
<211> 935
<212> PRT
<213> Pantoea ananatis

<400> 3
Met Gln Asn Ser Ala Met Lys Pro Trp Leu Asp Ser Ser Trp Leu Ala

1               5                   10                  15
Gly Ala Asn Gln Ser Tyr Ile Glu Gln Leu Tyr Glu Asp Phe Leu Thr
            20                  25                  30
Asp Pro Asp Ser Val Asp Ala Val Trp Arg Ser Met Phe Gln Gln Leu
        35                  40                  45
Pro Gly Thr Gly Val Lys Pro Glu Gln Phe His Ser Ala Thr Arg Glu
    50                  55                  60
Tyr Phe Arg Arg Leu Ala Lys Asp Ala Ser Arg Tyr Thr Ser Ser Val
65                  70                  75                  80
Thr Asp Pro Ala Thr Asn Ser Lys Gln Val Lys Val Leu Gln Leu Ile
                85                  90                  95
Asn Ala Phe Arg Phe Arg Gly His Gln Glu Ala Asn Leu Asp Pro Leu
            100                 105                 110
Gly Leu Trp Lys Gln Asp Arg Val Ala Asp Leu Asp Pro Ala Phe His
        115                 120                 125
Asp Leu Thr Asp Ala Asp Phe Gln Glu Ser Phe Asn Val Gly Ser Phe
    130                 135                 140
Ala Ile Gly Lys Glu Thr Met Lys Leu Ala Asp Leu Phe Asp Ala Leu
145                 150                 155                 160
Lys Gln Thr Tyr Cys Gly Ser Ile Gly Ala Glu Tyr Met His Ile Asn
                165                 170                 175
Asn Thr Glu Glu Lys Arg Trp Ile Gln Gln Arg Ile Glu Ser Gly Ala
            180                 185                 190
Ser Gln Thr Ser Phe Ser Gly Glu Glu Lys Lys Gly Phe Leu Lys Glu
        195                 200                 205
Leu Thr Ala Ala Glu Gly Leu Glu Lys Tyr Leu Gly Ala Lys Phe Pro
    210                 215                 220
Gly Ala Lys Arg Phe Ser Leu Glu Gly Gly Asp Ala Leu Val Pro Met
225                 230                 235                 240
Leu Arg Glu Met Ile Arg His Ala Gly Lys Ser Gly Thr Arg Glu Val
                245                 250                 255
Val Leu Gly Met Ala His Arg Gly Arg Leu Asn Val Leu Ile Asn Val
            260                 265                 270
Leu Gly Lys Lys Pro Gln Asp Leu Phe Asp Glu Phe Ser Gly Lys His
        275                 280                 285
Lys Glu His Leu Gly Thr Gly Asp Val Lys Tyr His Met Gly Phe Ser
    290                 295                 300
Ser Asp Ile Glu Thr Glu Gly Gly Leu Val His Leu Ala Leu Ala Phe
305                 310                 315                 320
Asn Pro Ser His Leu Glu Ile Val Ser Pro Val Val Met Gly Ser Val
                325                 330                 335
Arg Ala Arg Leu Asp Arg Leu Ala Glu Pro Val Ser Asn Lys Val Leu

            340                 345                 350
Pro Ile Thr Ile His Gly Asp Ala Ala Val Ile Gly Gln Gly Val Val
        355                 360                 365
Gln Glu Thr Leu Asn Met Ser Gln Ala Arg Gly Tyr Glu Val Gly Gly
    370                 375                 380
Thr Val Arg Ile Val Ile Asn Asn Gln Val Gly Phe Thr Thr Ser Asn
385                 390                 395                 400
Pro Lys Asp Ala Arg Ser Thr Pro Tyr Cys Thr Asp Ile Gly Lys Met
                405                 410                 415
Val Leu Ala Pro Ile Phe His Val Asn Ala Asp Asp Pro Glu Ala Val
            420                 425                 430
Ala Phe Val Thr Arg Leu Ala Leu Asp Tyr Arg Asn Thr Phe Lys Arg 435
440 445
Asp Val Phe Ile Asp Leu Val Cys Tyr Arg Arg His Gly His Asn Glu
    450                 455                 460
Ala Asp Glu Pro Ser Ala Thr Gln Pro Leu Met Tyr Gln Lys Ile Lys
465                 470                 475                 480
Lys His Pro Thr Pro Arg Lys Ile Tyr Ala Asp Arg Leu Glu Gly Glu
                485                 490                 495
Gly Val Ala Ser Gln Glu Asp Ala Thr Glu Met Val Asn Leu Tyr Arg
            500                 505                 510
Asp Ala Leu Asp Ala Gly Glu Cys Val Val Pro Glu Trp Arg Pro Met
        515                 520                 525
Ser Leu His Ser Phe Thr Trp Ser Pro Tyr Leu Asn His Glu Trp Asp
    530                 535                 540
Glu Pro Tyr Pro Ala Gln Val Asp Met Lys Arg Leu Lys Glu Leu Ala
545                 550                 555                 560
Leu Arg Ile Ser Gln Val Pro Glu Gln Ile Glu Val Gln Ser Arg Val
                565                 570                 575
Ala Lys Ile Tyr Asn Asp Arg Lys Leu Met Ala Glu Gly Glu Lys Ala
            580                 585                 590
Phe Asp Trp Gly Gly Ala Glu Asn Leu Ala Tyr Ala Thr Leu Val Asp
        595                 600                 605
Glu Gly Ile Pro Val Arg Leu Ser Gly Glu Asp Ser Gly Arg Gly Thr
    610                 615                 620
Phe Phe His Arg His Ala Val Val His Asn Gln Ala Asn Gly Ser Thr
625                 630                 635                 640
Tyr Thr Pro Leu His His Ile His Asn Ser Gln Gly Glu Phe Lys Val
                645                 650                 655
Trp Asp Ser Val Leu Ser Glu Glu Ala Val Leu Ala Phe Glu Tyr Gly
            660                 665                 670
Tyr Ala Thr Ala Glu Pro Arg Val Leu Thr Ile Trp Glu Ala Gln Phe

675                 680                 685
Gly Asp Phe Ala Asn Gly Ala Gln Val Val Ile Asp Gln Phe Ile Ser
    690                 695                 700
Ser Gly Glu Gln Lys Trp Gly Arg Met Cys Gly Leu Val Met Leu Leu
705                 710                 715                 720
Pro His Gly Tyr Glu Gly Gln Gly Pro Glu His Ser Ser Ala Arg Leu
                725                 730                 735
Glu Arg Tyr Leu Gln Leu Cys Ala Glu Gln Asn Met Gln Val Cys Val
            740                 745                 750
Pro Ser Thr Pro Ala Gln Val Tyr His Met Leu Arg Arg Gln Ala Leu
        755                 760                 765
Arg Gly Met Arg Arg Pro Leu Val Val Met Ser Pro Lys Ser Leu Leu
    770                 775                 780
Arg His Pro Leu Ala Ile Ser Ser Leu Asp Glu Leu Ala Asn Gly Ser
785                 790                 795                 800
Phe Gln Pro Ala Ile Gly Glu Ile Asp Asp Leu Asp Pro Gln Gly Val
                805                 810                 815
Lys Arg Val Val Leu Cys Ser Gly Lys Val Tyr Tyr Asp Leu Leu Glu
            820                 825                 830
Gln Arg Arg Lys Asp Glu Lys Thr Asp Val Ala Ile Val Arg Ile Glu
        835                 840                 845
Gln Leu Tyr Pro Phe Pro His Gln Ala Val Gln Glu Ala Leu Lys Ala
    850                 855                 860
Tyr Ser His Val Gln Asp Phe Val Trp Cys Gln Glu Glu Pro Leu Asn
865                 870                 875                 880
Gln Gly Ala Trp Tyr Cys Ser Gln His His Phe Arg Asp Val Val Pro
                885                 890                 895
Phe Gly Ala Thr Leu Arg Tyr Ala Gly Arg Pro Ala Ser Ala Ser Pro
            900                 905                 910
Ala Val Gly Tyr Met Ser Val His Gln Gln Gln Gln Gln Asp Leu Val
        915                 920                 925
Asn Asp Ala Leu Asn Val Asn
    930                 935

<210> 4
<211> 407
<212> PRT
<213> Pantoea ananatis

<400> 4
Met Ser Ser Val Asp Ile Leu Val Pro Asp Leu Pro Glu Ser Val Ala
1               5                   10                  15

Asp Ala Thr Val Ala Thr Trp His Lys Lys Pro Gly Asp Ala Val Ser
            20                  25                  30
Arg Asp Glu Val Ile Val Glu Ile Glu Thr Asp Lys Val Val Leu Glu
        35                  40                  45
Val Pro Ala Ser Ala Asp Gly Val Leu Glu Ala Val Leu Glu Asp Glu
    50                  55                  60
Gly Ala Thr Val Thr Ser Arg Gln Ile Leu Gly Arg Leu Lys Glu Gly
65                  70                  75                  80
Asn Ser Ala Gly Lys Glu Ser Ser Ala Lys Ala Glu Ser Asn Asp Thr
                85                  90                  95
Thr Pro Ala Gln Arg Gln Thr Ala Ser Leu Glu Glu Glu Ser Ser Asp
            100                 105                 110
Ala Leu Ser Pro Ala Ile Arg Arg Leu Ile Ala Glu His Asn Leu Asp
        115                 120                 125
Ala Ala Gln Ile Lys Gly Thr Gly Val Gly Gly Arg Leu Thr Arg Glu
    130                 135                 140
Asp Val Glu Lys His Leu Ala Asn Lys Pro Gln Ala Glu Lys Ala Ala
145                 150                 155                 160
Ala Pro Ala Ala Gly Ala Ala Thr Ala Gln Gln Pro Val Ala Asn Arg
                165                 170                 175
Ser Glu Lys Arg Val Pro Met Thr Arg Leu Arg Lys Arg Val Ala Glu
            180                 185                 190
Arg Leu Leu Glu Ala Lys Asn Ser Thr Ala Met Leu Thr Thr Phe Asn
        195                 200                 205
Glu Ile Asn Met Lys Pro Ile Met Asp Leu Arg Lys Gln Tyr Gly Asp
    210                 215                 220
Ala Phe Glu Lys Arg His Gly Val Arg Leu Gly Phe Met Ser Phe Tyr
225                 230                 235                 240
Ile Lys Ala Val Val Glu Ala Leu Lys Arg Tyr Pro Glu Val Asn Ala
                245                 250                 255
Ser Ile Asp Gly Glu Asp Val Val Tyr His Asn Tyr Phe Asp Val Ser
            260                 265                 270
Ile Ala Val Ser Thr Pro Arg Gly Leu Val Thr Pro Val Leu Arg Asp
        275                 280                 285
Val Asp Ala Leu Ser Met Ala Asp Ile Glu Lys Lys Ile Lys Glu Leu
    290                 295                 300
Ala Val Lys Gly Arg Asp Gly Lys Leu Thr Val Asp Asp Leu Thr Gly
305                 310                 315                 320
Gly Asn Phe Thr Ile Thr Asn Gly Gly Val Phe Gly Ser Leu Met Ser
                325                 330                 335
Thr Pro Ile Ile Asn Pro Pro Gln Ser Ala Ile Leu Gly Met His Ala
            340                 345                 350

Ile Lys Asp Arg Pro Met Ala Val Asn Gly Gln Val Val Ile Leu Pro
        355                 360                 365
Met Met Tyr Leu Ala Leu Ser Tyr Asp His Arg Leu Ile Asp Gly Arg
    370                 375                 380
Glu Ser Val Gly Tyr Leu Val Ala Val Lys Glu Met Leu Glu Asp Pro
385                 390                 395                 400
Ala Arg Leu Leu Leu Asp Val
                405

<210> 5
<211> 40
<212> PRT
<213> Pantoea ananatis

<400> 5
Met Asn Leu His Glu Tyr Gln Ala Lys Gln Leu Phe Ala Arg Tyr Gly
  1               5                  10                  15
Met Pro Ala Pro Thr Gly Tyr Ala Cys Thr Thr Pro Arg Glu Ala Glu
             20                  25                  30
Glu Ala Ala Ser Lys Ile Gly Ala
         35                  40

<210> 6
<211> 30
<212> DNA
<213> Artificial Sequence

<220>
<223> Description of Artificial Sequence: primer

<400> 6
gtcgacaata gccygaatct gttctggtcg                                  30

<210> 7
<211> 30
<212> DNA
<213> Artificial Sequence

<220>
<223> Description of Artificial Sequence: primer

<400> 7

aagcttatcg acgctcccct ccccaccgtt                                    30

<210> 8
<211> 16214
<212> DNA
<213> Artificial Sequence

<220>
<223> Description of Artificial Sequence: plasmid RSFCPG

<400> 8
gaattccgcc agaaccttca tcagcagcat aaacaggtgc agtgaacagc agagatacgg    60
ccagtgcggc caatgttttt tgtcctttaa acataacaga gtcctttaag gatatagaat   120
aggggtatag ctacgccaga atatcgtatt tgattattgc tagtttttag ttttgcttaa   180
aaaatattgt tagttttatt aaattggaaa actaaattat tggtatcatg aattgttgta   240
tgatgataaa tatagggggg atatgataga cgtcattttc atagggttat aaaatgcgac   300
taccatgaag tttttaattc aaagtattgg gttgctgata atttgagctg ttctattctt   360
tttaaatatc tatataggtc tgttaatgga ttttattttt acaagttttt tgtgtttagg   420
catataaaaa tcaagcccgc catatgaacg gcgggttaaa atatttacaa cttagcaatc   480
gaaccattaa cgcttgatat cgcttttaaa gtcgcgtttt tcatatcctg tatacagctg   540
acgcggacgg gcaatcttca taccgtcact gtgcatttcg ctccagtggg cgatccagcc   600
aacggtacgt gccattgcga aaatgacggt gaacatggaa gacggaatac ccatcgcttt   660
caggatgata ccagagtaga aatcgacgtt cgggtacagt ttcttctcga taaagtacgg   720
gtcgttcagc gcgatgtttt ccagctccat agccacttcc agcaggtcat ccttcgtgcc   780
cagctctttc agcacttcat ggcaggtttc acgcattacg gtggcgcgcg ggtcgtaatt   840
tttgtacacg cggtgaccga agcccatcag gcggaaagaa tcatttttgt ctttcgcacg   900
acgaaaaaat tccggaatgt gtttaacgga gctgatttct tccagcattt tcagcgccgc   960
ttcgttagca ccgccgtgcg caggtcccca cagtgaagca atacctgctg cgatacaggc  1020
aaacgggttc gcacccgaag agccagcggt acgcacggtg gaggtagagg cgttctgttc  1080
atggtcagcg tgcaggatca gaatacggtc catagcacgt tccagaatcg gattaacttc  1140
atacggttcg cacggcgtgg agaacatcat attcaggaag ttaccggcgt aggagagatc  1200
gttgcgcggg taaacaaatg gctgaccaat ggaatacttg taacacatcg cggccatggt  1260
cggcattttc gacagcaggc ggaacgcggc aatttcacgg tgacgaggat tgttaacatc  1320
cagcgagtcg tgatagaacg ccgccagcgc gccggtaata ccacacatga ctgccattgg  1380
atgcgagtcg cgacggaaag catggaacag acgggtaatc tgctcgtgga tcatggtatg  1440
acgggtcacc gtagttttaa attcgtcata ctgttcctga gtcggttttt caccattcag  1500
caggatgtaa caaacttcca ggtagttaga atcggtcgcc agctgatcga tcgggaaacc  1560
gcggtgcagc aaaatacctt catcaccatc aataaaagta attttagatt cgcaggatgc  1620
ggttgaagtg aagcctgggt caaaggtgaa cacacctttt gaaccgagag tacggatatc  1680
aataacatct tgacccagcg tgcctttcag cacatccagt tcaacagctg tatccccgtt  1740
gagggtgagt tttgcttttg tatcagccat ttaaggtctc cttagcgcct tattgcgtaa  1800
gactgccgga acttaaattt gccttcgcac atcaacctgg ctttacccgt tttttatttg  1860

gctcgccgct ctgtgaaaga ggggaaaacc tgggtacaga gctctgggcg cttgcaggta 1920
aaggatccat tgatgacgaa taaatggcga atcaagtact tagcaatccg aattattaaa 1980
cttgtctacc actaataact gtcccgaatg aattggtcaa tactccacac tgttacataa 2040
gttaatctta ggtgaaatac cgacttcata acttttacgc attatatgct tttcctggta 2100
atgtttgtaa caactttgtt gaatgattgt caaattagat gattaaaaat taaataaatg 2160
ttgttatcgt gacctggatc actgttcagg ataaaacccg acaaactata tgtaggttaa 2220
ttgtaatgat tttgtgaaca gcctatactg ccgccagtct ccggaacacc ctgcaatccc 2280
gagccaccca gcgttgtaac gtgtcgtttt cgcatctgga agcagtgttt tgcatgacgc 2340
gcagttatag aaaggacgct gtctgacccg caagcagacc ggaggaagga aatcccgacg 2400
tcggggatcc tctagagctt tagcgtctga ggttatcgca atttggttat gagattactc 2460
tcgttattaa tttgctttcc tgggtcattt ttttcttgct taccgtcaca ttcttgatgg 2520
tatagtcgaa aactgcaaaa gcacatgaca taaacaacat aagcacaatc gtattaatat 2580
ataagggttt tatatctatg gatcagacat attctctgga gtcattcctc aaccatgtcc 2640
aaaagcgcga cccgaatcaa accgagttcg cgcaagccgt tcgtgaagta atgaccacac 2700
tctggccttt tcttgaacaa aatccaaaat atcgccagat gtcattactg gagcgtctgg 2760
ttgaaccgga gcgcgtgatc cagtttcgcg tggtatgggt tgatgatcgc aaccagatac 2820
aggtcaaccg tgcatggcgt gtgcagttca gctctgccat cggcccgtac aaaggcggta 2880
tgcgcttcca tccgtcagtt aacctttcca ttctcaaatt cctcggcttt gaacaaacct 2940
tcaaaaatgc cctgactact ctgccgatgg gcggtggtaa aggcggcagc gatttcgatc 3000
cgaaaggaaa aagcgaaggt gaagtgatgc gtttttgcca ggcgctgatg actgaactgt 3060
atcgccacct gggcgcggat accgacgttc cggcaggtga tatcggggtt ggtggtcgtg 3120
aagtcggctt tatggcgggg atgatgaaaa agctctccaa caataccgcc tgcgtcttca 3180
ccggtaaggg cctttcattt ggcggcagtc ttattcgccc ggaagctacc ggctacggtc 3240
tggtttattt cacagaagca atgctaaaac gccacggtat gggttttgaa gggatgcgcg 3300
tttccgtttc tggctccggc aacgtcgccc agtacgctat cgaaaaagcg atggaatttg 3360
gtgctcgtgt gatcactgcg tcagactcca gcggcactgt agttgatgaa agcggattca 3420
cgaaagagaa actggcacgt cttatcgaaa tcaaagccag ccgcgatggt cgagtggcag 3480
attacgccaa agaatttggt ctggtctatc tcgaaggcca acagccgtgg tctctaccgg 3540
ttgatatcgc cctgccttgc gccacccaga atgaactgga tgttgacgcc gcgcatcagc 3600
ttatcgctaa tggcgttaaa gccgtcgccg aaggggcaaa tatgccgacc accatcgaag 3660
cgactgaact gttccagcag gcaggcgtac tatttgcacc gggtaaagcg gctaatgctg 3720
gtggcgtcgc tacatcgggc ctggaaatgc cacaaaacgc tgcgcgcctg ggctggaaag 3780
ccgagaaagt tgacgcacgt ttgcatcaca tcatgctgga tatccaccat gcctgtgttg 3840
agcatggtgg tgaaggtgag caaaccaact acgtgcaggg cgcgaacatt gccggttttg 3900
tgaaggttgc cgatgcgatg ctggcgcagg gtgtgattta agttgtaaat gcctgatggc 3960
gctacgctta tcaggcctac aaatgggcac aattcattgc agttacgctc taatgtaggc 4020
cgggcaagcg cagcgccccc ggcaaaattt caggcgttta tgagtattta acggatgatg 4080
ctccccacgg aacatttctt atgggccaac ggcatttctt actgtagtgc tcccaaaact 4140
gcttgtcgta acgataacac gcttcaagtt cagcatccgt taactttctg cggactcacg 4200
cgcgcagcac tatgccagta aagaaatccc atttgactat ttttttgata atcttcttcg 4260
ctttcgaaca actcgtgcgc ctttcgagaa gctagagtcg actcgccaat caccagcact 4320
aaagtgcgcg gttcgttacc cgattcatct ttgaaattag ccagtggcgg caaggcatta 4380

ttttcattca gtaactttgt tagcgagttt agttgctgac gatactgata atagccggtc 4440
aggaattgcc acggtgcggc aggctccata cgcgaggcca ggttatccaa cgttttctca 4500
aacggcttgt ttttgataaa cgtattcatg gcgatcggat gcagaatcaa gccataaagc 4560
agggcaaaag agacaacata acgccacggc tttggaatat agaccgggcg caggcgtgtc 4620
cacagcagaa ctgccaccgc cgtataggcc agcgcgataa gcacaatttt caggctgaaa 4680
tactggctta aatactcgct ggcttcgttg gtgttggttt cgaacatcac aaacagaacg 4740
ctctgcgaga actcctgacc gtagatgacg tagtagcaca gcgccgccag agaggccgcc 4800
catagcacca cgccgattac tgcggcaata attttaatcc gcttcggaaa gaggaatacc 4860
gggatcaacc acagcgaact gaataacagc gagtcgcgaa tgccgttagt gccactataa 4920
ccactgatgt aaataatggc ctgtagcaga gtagagaaaa accaaaagta gagcagtgcc 4980
caacccaggg ctttccagct aaaaagaggt ttagcctgga cttctgtgga atgcatagta 5040
agaacctgtc ttgaaaaaat atcgccgaat gtaacgacaa ttccttaagg atatctgaag 5100
gtatattcag aatttgaata aaatgcagac agaaatatat tgaaaacgag ggtgttagaa 5160
cagaagtatt tcagaaaacc ctcgcgcaaa agcacgaggg tttgcagaag aggaagatta 5220
gccggtatta cgcatacctg ccgcaatccc ggcaatagtg accattaacg cttgttcgac 5280
gcgaggatcc ggttcctggc cttctttttc tgcctggcgg gagcggtgca gcaactcggc 5340
ctgcaatacg ttcagcgggt cggtgtaaat attccgtagc tgaatagact ctgcaatcca 5400
cggcagatcg gccatcagat gggaatcgtt ggcaatcgcc agcaccactt tgatgtcttc 5460
ttcttgcagg ttgcgtaact ctttacctaa cggccacagt gctttgtcta ccaggcgttg 5520
gtcatagtat tccgccagcc acaggtctgc tttggcgaag accatctcca gcatgccgag 5580
acgcgtcgag aagaatggcc aatcgcggca catagcctcc agctcgctct gtttgccgtc 5640
ttcgaccact ttttgcagcg ccgtacctgc acccagccag gcggggagca tcagacggtt 5700
ttgcgtccag gcgaagatcc acggaatggc gcgtagtgac tcgacgccgc cggttgggcg 5760
acgtttcgcc ggacgtgaac ccaacggcag tttgcccagt tcttgttccg gcgtagcgga 5820
gcggaagtaa ggcacaaaat ctttgttttc acgtacgtag ccgcggtaga catcgcagga 5880
gatgactgac agttcatcca taatgcgacg ccagctctct ttcggctccg gcggtggcag 5940
caggttggct tccagaatcg ccccggtata aagcgacagg ctgctgacgg tgatttctgg 6000
cagaccatat ttaaagcgga tcatctcgcc ctgttcggtt acgcgcaggc cgcctttcag 6060
gcttcctggc ggttgtgaca gcagcgccgc atgagcaggt gcgccgccgc gaccaatgga 6120
accgccgcga ccgtggaaca acgtcagctc aatacccgct ttttcgcagg ttttgattaa 6180
tgcatcctgt gcctgatatt gcgcccagga agctgccatc actcccgcat cttttgctga 6240
gtcggaatag ccaatcatca ccatctgttt gccctgaatc aggccacgat accagtcaat 6300
attgagcagc tgggtcatga catcgttggc gttgttcaga tcatcgaggg tttcaaacag 6360
cggagcaacc ggcatcgcaa acccgatacc cgcttctttc agcagcaggt ggacagccag 6420
tacgtcggac ggcgttttcg ccatcgagat cacgtaggcg gcaatggagc cttgcggtgc 6480
ttcggcaatc acctggcagg tatcgagcac ttcgcgcgtt tcggcgcttg gttgccagtt 6540
gcgcggcaga agcggacgtt tggagttcag ttcgcggatc aggaacgcct gtttgtcggc 6600
ctctgaccag ctttcgtagt cgccgatacc gaggtagcgg gtcagctcgc ccagcgcttc 6660
ggtatgacgc gtgctctcct gacggatatc aatacggacc agcggtacgc cgaaacattt 6720
cacgcggcgc agggtgtcga gcagatcgcc gttggcgata atacccatgc cacacgcctg 6780
aagtgactgg tagcaagcgt agagcggttc ccacagttct tcgttttgtg tcagcaggcc 6840
ttctggtttt ggcagttctt cgcctttcag gcgcgcttcc agccatgcct gtgtcgccat 6900

caggcgagaa cgcaggtttt tcatcagata gcgatacggt tctgcggcac cttcttcgcc 6960
aaccagcgcc agcagttcag gggtcgcttc aaccatcgac agttcagaaa ccagcacctg 7020
aatatctttc aggaacaaat cggtggcttt ccagcggctg agtagcagga cgtggcgggt 7080
gatatcggca gtgacgttcg ggttgccgtc gcggtcgccg cccatccacg aagtaaaacg 7140
gaccggaaca aattcgacgg gcagtttgta gccgaggttc tcttccagtt gttcgttcag 7200
ttcgcgcagg taatttggta cgccttgcca caggctgttt tccactacgg caaagcccca 7260
tttggcttca tctaccgggc ttggacgcag cttacggatt tcatcggtat gccatgactg 7320
ggcgatcaac tggcgcaggc gacgcatcag ctggttgtgt tcgtagtcag cgatatcttt 7380
gttatcgagc tgttttaaac aggcgttcac ttccaccatt ttgtggatca gtgtacgacg 7440
ggtaatttcg gttgggtgag ccgtgaggac cagttccagc gacagcgatt ccactgcttt 7500
tttgatggtg tcttcgctca gttccggctg gtttttcagt ttacgcaggg tgcgggcgat 7560
cacttccggg ttgctggcag cttcgccttt cggcgaaatg ctgtggtatt gctcggcggt 7620
gttggccagg ttcaggaact gactaaacgc acgcgcaacg ggcagcagct cgtcgttcga 7680
caaattttgt aaggtggtga gcaactcctg gcggttagca tcattgccag cgcgtgaaga 7740
tttcgacaac ttacggatag tttctacgcg ttcaagaatg tgttctccca acgcatcctt 7800
gatggtttct cccagcactt tgccgagcat actgacatta ctacgcaatg cggaatattg 7860
ttcgttcata ttaccccaga caccccatct tatcgtttga tagccctgta tccttcacgt 7920
cgcattggcg cgaatatgct cgggctttgc ttttcgtcgt cttttataaa gccacgtaaa 7980
agcggtgacg tcaaatgctg cgaaatcgct tcagcaaacg aataaatagc aggaatttac 8040
gtcattaaat tcacgacgct ttaaataagc gtaacttatg gaaatgttaa aaaatcgccc 8100
caagtaacac caaaggtgta ggtcggataa gatgcgcaag tatcgcatcc gacattattg 8160
cggcactgga gtttggcaac agtgccggat gcggcgcgag cgccttatcc ggcctacagt 8220
tgggcatcgt ttgagtcact gtcggtcgga taagatgcgc aagtatcgca tccgacatta 8280
ttgcggcact ggagtttggc aacagtgccg gatgcggcgc gagcgcctta tccggcctac 8340
ggttgggcat cgtttgagtc actgtaggtc ggataagatg cgcaagcatc gcatccgaca 8400
ttattgcggc actggagttt ggcaacagcg ccggatgcgg cgcgagcgcc ttatccggcc 8460
tacgttttaa tgccagcaaa aatggtgaat tacctgggtt atcagttcgc gggtgggctt 8520
gataaaccgt gtttccagat attcatcagg ttgatgagcc tgattaattg agccaggccc 8580
caacaccagc gtcgggcata acgtttgaat aaacggcgct tcggtacagt agttcaccac 8640
ttcggttttt gctccgagca atttctcaac cacttcaacc agttgatgat tcggtgggca 8700
ttcatagcca gggatcggcg gatgcagctc gtcgacctgc aggagcagaa gagcatacat 8760
ctggaagcaa agccaggaaa gcggcctatg gagctgtgcg gcagcgctca gtaggcaatt 8820
tttcaaaata ttgttaagcc ttttctgagc atggtatttt tcatggtatt accaattagc 8880
aggaaaataa gccattgaat ataaaagata aaaatgtctt gtttacaata gagtgggggg 8940
ggtcagcctg ccgccttggg ccgggtgatg tcgtacttgc ccgccgcgaa ctcggttacc 9000
gtccagccca gcgcgaccag ctccggcaac gcctcgcgca cccgctggcg gcgcttgcgc 9060
atggtcgaac cactggcctc tgacggccag acatagccgc acaaggtatc tatggaagcc 9120
ttgccggttt tgccggggtc gatccagcca cacagccgct ggtgcagcag gcgggcggtt 9180
tcgctgtcca gcgcccgcac ctcgtccatg ctgatgcgca catgctggcc gccacccatg 9240
acggcctgcg cgatcaaggg gttcagggcc acgtacaggc gcccgtccgc ctcgtcgctg 9300
gcgtactccg acagcagccg aaacccctgc cgcttgcggc cattctgggc gatgatggat 9360
accttccaaa ggcgctcgat gcagtcctgt atgtgcttga gcgccccacc actatcgacc 9420

tctgccccga tttcctttgc cagcgcccga tagctacctt tgaccacatg gcattcagcg 9480
gtgacggcct cccacttggg ttccaggaac agccggagct gccgtccgcc ttcggtcttg 9540
ggttccgggc caagcactag gccattaggc ccagccatgg ccaccagccc ttgcaggatg 9600
cgcagatcat cagcgcccag cggctccggg ccgctgaact cgatccgctt gccgtcgccg 9660
tagtcatacg tcacgtccag cttgctgcgc ttgcgctcgc cccgcttgag ggcacggaac 9720
aggccggggg ccagacagtg cgcccggtcg tgccggacgt ggctgaggct gtgcttgttc 9780
ttaggcttca ccacggggca cccccttgct cttgcgctgc ctctccagca cggcgggctt 9840
gagcaccccg ccgtcatgcc gcctgaacca ccgatcagcg aacggtgcgc catagttggc 9900
cttgctcaca ccgaagcgga cgaagaaccg gcgctggtcg tcgtccacac cccattcctc 9960
ggcctcggcg ctggtcatgc tcgacaggta ggactgccag cggatgttat cgaccagtac 10020
cgagctgccc cggctggcct gctgctggtc gcctgcgccc atcatggccg cgcccttgct 10080
ggcatggtgc aggaacacga tagagcaccc ggtatcggcg gcgatggcct ccatgcgacc 10140
gatgacctgg gccatggggc cgctggcgtt ttcttcctcg atgtggaacc ggcgcagcgt 10200
gtccagcacc atcaggcggc ggccctcggc ggcgcgcttg aggccgtcga accactccgg 10260
ggccatgatg ttgggcaggc tgccgatcag cggctggatc agcaggccgt cagccacggc 10320
ttgccgttcc tcggcgctga ggtgcgcccc aagggcgtgc aggcggtgat gaatggcggt 10380
gggcgggtct tcggcgggca ggtagatcac cgggccggtg ggcagttcgc ccacctccag 10440
cagatccggc ccgcctgcaa tctgtgcggc cagttgcagg gccagcatgg atttaccggc 10500
accaccgggc gacaccagcg ccccgaccgt accggccacc atgttgggca aaacgtagtc 10560
cagcggtggc ggcgctgctg cgaacgcctc cagaatattg ataggcttat gggtagccat 10620
tgattgcctc ctttgcaggc agttggtggt taggcgctgg cggggtcact acccccgccc 10680
tgcgccgctc tgagttcttc caggcactcg cgcagcgcct cgtattcgtc gtcggtcagc 10740
cagaacttgc gctgacgcat ccctttggcc ttcatgcgct cggcatatcg cgcttggcgt 10800
acagcgtcag ggctggccag caggtcgccg gtctgcttgt ccttttggtc tttcatatca 10860
gtcaccgaga aacttgccgg ggccgaaagg cttgtcttcg cggaacaagg acaaggtgca 10920
gccgtcaagg ttaaggctgg ccatatcagc gactgaaaag cggccagcct cggccttgtt 10980
tgacgtataa ccaaagccac cgggcaacca atagcccttg tcacttttga tcaggtagac 11040
cgaccctgaa gcgctttttt cgtattccat aaaaccccct tctgtgcgtg agtactcata 11100
gtataacagg cgtgagtacc aacgcaagca ctacatgctg aaatctggcc cgcccctgtc 11160
catgcctcgc tggcggggtg ccggtgcccg tgccagctcg gcccgcgcaa gctggacgct 11220
gggcagaccc atgaccttgc tgacggtgcg ctcgatgtaa tccgcttcgt ggccgggctt 11280
gcgctctgcc agcgctgggc tggcctcggc catggccttg ccgatttcct cggcactgcg 11340
gccccggctg gccagcttct gcgcggcgat aaagtcgcac ttgctgaggt catcaccgaa 11400
gcgcttgacc agcccggcca tctcgctgcg gtactcgtcc agcgccgtgc gccggtggcg 11460
gctaagctgc cgctcgggca gttcgaggct ggccagcctg cgggccttct cctgctgccg 11520
ctgggcctgc tcgatctgct ggccagcctg ctgcaccagc gccgggccag cggtggcggt 11580
cttgcccttg gattcacgca gcagcaccca cggctgataa ccggcgcggg tggtgtgctt 11640
gtccttgcgg ttggtgaagc ccgccaagcg gccatagtgg cggctgtcgg cgctggccgg 11700
gtcggcgtcg tactcgctgg ccagcgtccg ggcaatctgc ccccgaagtt caccgcctgc 11760
ggcgtcggcc accttgaccc atgcctgata gttcttcggg ctggtttcca ctaccagggc 11820
aggctcccgg ccctcggctt tcatgtcatc caggtcaaac tcgctgaggt cgtccaccag 11880
caccagacca tgccgctcct gctcggcggg cctgatatac acgtcattgc cctgggcatt 11940

catccgcttg agccatggcg tgttctggag cacttcggcg gctgaccatt cccggttcat 12000
catctggccg gtggtggcgt ccctgacgcc gatatcgaag cgctcacagc ccatggcctt 12060
gagctgtcgg cctatggcct gcaaagtcct gtcgttcttc atcgggccac caagcgcagc 12120
cagatcgagc cgtcctcggt tgtcagtggc gtcaggtcga gcaagagcaa cgatgcgatc 12180
agcagcacca ccgtaggcat catggaagcc agcatcacgg ttagccatag cttccagtgc 12240
cacccccgcg acgcgctccg ggcgctctgc gcggcgctgc tcacctcggc ggctacctcc 12300
cgcaactctt tggccagctc cacccatgcc gcccctgtct ggcgctgggc tttcagccac 12360
tccgccgcct gcgcctcgct ggcctgctgg gtctggctca tgacctgccg ggcttcgtcg 12420
gccagtgtcg ccatgctctg ggccagcggt tcgatctgct ccgctaactc gttgatgcct 12480
ctggatttct tcactctgtc gattgcgttc atggtctatt gcctcccggt attcctgtaa 12540
gtcgatgatc tgggcgttgg cggtgtcgat gttcagggcc acgtctgccc ggtcggtgcg 12600
gatgccccgg ccttccatct ccaccacgtt cggccccagg tgaacaccgg gcaggcgctc 12660
gatgccctgc gcctcaagtg ttctgtggtc aatgcgggcg tcgtggccag cccgctctaa 12720
tgcccggttg gcatggtcgg cccatgcctc gcgggtctgc tcaagccatg ccttgggctt 12780
gagcgcttcg gtcttctgtg ccccgccctt ctccggggtc ttgccgttgt accgcttgaa 12840
ccactgagcg gcgggccgct cgatgccgtc attgatccgc tcggagatca tcaggtggca 12900
gtgcgggttc tcgccgccac cggcatggat ggccagcgta tacggcaggc gctcggcacc 12960
ggtcaggtgc tgggcgaact cggacgccag cgccttctgc tggtcgaggg tcagctcgac 13020
cggcagggca aattcgacct ccttgaacag ccgcccattg gcgcgttcat acaggtcggc 13080
agcatcccag tagtcggcgg gccgctcgac gaactccggc atgtgcccgg attcggcgtg 13140
caagacttca tccatgtcgc gggcatactt gccttcgcgc tggatgtagt cggccttggc 13200
cctggccgat tggccgcccg acctgctgcc ggttttcgcc gtaaggtgat aaatcgccat 13260
gctgcctcgc tgttgctttt gcttttcggc tccatgcaat ggccctcgga gagcgcaccg 13320
cccgaagggt ggccgttagg ccagtttctc gaagagaaac cggtaagtgc gccctcccct 13380
acaaagtagg gtcgggattg ccgccgctgt gcctccatga tagcctacga gacagcacat 13440
taacaatggg gtgtcaagat ggttaagggg agcaacaagg cggcggatcg gctggccaag 13500
ctcgaagaac aacgagcgcg aatcaatgcc gaaattcagc gggtgcgggc aagggaacag 13560
cagcaagagc gcaagaacga aacaaggcgc aaggtgctgg tgggggccat gattttggcc 13620
aaggtgaaca gcagcgagtg gccggaggat cggctcatgg cggcaatgga tgcgtacctt 13680
gaacgcgacc acgaccgcgc cttgttcggt ctgccgccac gccagaagga tgagccgggc 13740
tgaatgatcg accgagacag gccctgcggg gctgcacacg cgcccccacc cttcgggtag 13800
ggggaaaggc cgctaaagcg gctaaaagcg ctccagcgta tttctgcggg gtttggtgtg 13860
gggtttagcg ggctttgccc gcctttcccc ctgccgcgca gcggtggggc ggtgtgtagc 13920
ctagcgcagc gaatagacca gctatccggc ctctggccgg gcatattggg caagggcagc 13980
agcgccccac aagggcgctg ataaccgcgc ctagtggatt attcttagat aatcatggat 14040
ggatttttcc aacaccccgc cagcccccgc ccctgctggg tttgcaggtt tgggggcgtg 14100
acagttattg caggggttcg tgacagttat tgcagggggg cgtgacagtt attgcagggg 14160
ttcgtgacag ttagtacggg agtgacgggc actggctggc aatgtctagc aacggcaggc 14220
atttcggctg agggtaaaag aactttccgc taagcgatag actgtatgta aacacagtat 14280
tgcaaggacg cggaacatgc ctcatgtggc ggccaggacg gccagccggg atcgggatac 14340
tggtcgttac cagagccacc gacccgagca aacccttctc tatcagatcg ttgacgagta 14400
ttacccggca ttcgctgcgc ttatggcaga gcagggaaag gaattgccgg gctatgtgca 14460

acgggaattt gaagaatttc tccaatgcgg gcggctggag catggctttc tacgggttcg 14520
ctgcgagtct tgccacgccg agcacctggt cgctttcagc tgtaagcgtc gcggtttctg 14580
cccgagctgt ggggcgcggc ggatggccga aagtgccgcc ttgctggttg atgaagtact 14640
gcctgaacaa cccatgcgtc agtgggtgtt gagcttcccg tttcagctgc gtttcctgtt 14700
tggggtcgtt tgcgggaagg ggcggaatcc tacgctaagg ctttggccag cgatattctc 14760
cggtgagatt gatgtgttcc caggggatag gagaagtcgc ttgatatcta gtatgacgtc 14820
tgtcgcacct gcttgatcgc ggcccaaggg ttggtttgcg cattcacagt tctccgcaag 14880
aattgattgg ctccaattct tggagtggtg aatccgttag cgaggtgccg ccggcttcca 14940
ttcaggtcga ggtggcccgg ctccatgcac cgcgacgcaa cgcggggagg cagacaaggt 15000
atagggcggc gcctacaatc catgccaacc cgttccatgt gctcgccgag gcggcataaa 15060
tcgccgtgac gatcagcggt ccagtgatcg aagttaggct ggtaagagcc gcgagcgatc 15120
cttgaagctg tccctgatgg tcgtcatcta cctgcctgga cagcatggcc tgcaacgcgg 15180
gcatcccgat gccgccggaa gcgagaagaa tcataatggg gaaggccatc cagcctcgcg 15240
tcgcgaacgc cagcaagacg tagcccagcg cgtcggccgc catgccggcg ataatggcct 15300
gcttctcgcc gaaacgtttg gtggcgggac cagtgacgaa ggcttgagcg agggcgtgca 15360
agattccgaa taccgcaagc gacaggccga tcatcgtcgc gctccagcga aagcggtcct 15420
cgccgaaaat gacccagagc gctgccggca cctgtcctac gagttgcatg ataaagaaga 15480
cagtcataag tgcggcgacg atagtcatgc cccgcgccca ccggaaggag ctgactgggt 15540
tgaaggctct caagggcatc ggtcgacgct ctcccttatg cgactcctgc attaggaagc 15600
agcccagtag taggttgagg ccgttgagca ccgccgccgc aaggaatggt gcatgcaagg 15660
agatggcgcc caacagtccc ccggccacgg ggcctgccac catacccacg ccgaaacaag 15720
cgctcatgag cccgaagtgg cgagcccgat cttccccatc ggtgatgtcg gcgatatagg 15780
cgccagcaac cgcacctgtg gcgccggtga tgccggccac gatgcgtccg gcgtagagga 15840
tccacaggac gggtgtggtc gccatgatcg cgtagtcgat agtggctcca agtagcgaag 15900
cgagcaggac tgggcggcgg ccaaagcggt cggacagtgc tccgagaacg ggtgcgcata 15960
gaaattgcat caacgcatat agcgctagca gcacgccata gtgactggcg atgctgtcgg 16020
aatggacgat atcccgcaag aggcccggca gtaccggcat aaccaagcct atgcctacag 16080
catccagggt gacggtgccg aggatgacga tgagcgcatt gttagatttc atacacggtg 16140
cctgactgcg ttagcaattt aactgtgata aactaccgca ttaaagctta tcgatgataa 16200
gctgtcaaac atga 16214

<210> 9
<211> 427
<212> PRT
<213> Pantoea ananatis

<400> 9
Met Ala Asp Thr Lys Ala Lys Leu Thr Leu Asn Gly Asp Thr Ala Val
1               5                   10                  15
Glu Leu Asp Val Leu Lys Gly Thr Leu Gly Gln Asp Val Ile Asp Ile
            20                  25                  30
Arg Thr Leu Gly Ser Lys Gly Val Phe Thr Phe Asp Pro Gly Phe Thr

        35                  40                  45
Ser Thr Ala Ser Cys Glu Ser Lys Ile Thr Phe Ile Asp Gly Asp Glu
    50                  55                  60
Gly Ile Leu Leu His Arg Gly Phe Pro Ile Asp Gln Leu Ala Thr Asp
65                  70                  75                  80
Ser Asn Tyr Leu Glu Val Cys Tyr Ile Leu Leu Asn Gly Glu Lys Pro
                85                  90                  95
Thr Gln Glu Gln Tyr Asp Glu Phe Lys Thr Thr Val Thr Arg His Thr
            100                 105                 110
Met Ile His Glu Gln Ile Thr Arg Leu Phe His Ala Phe Arg Arg Asp
        115                 120                 125
Ser His Pro Met Ala Val Met Cys Gly Ile Thr Gly Ala Leu Ala Ala
    130                 135                 140
Phe Tyr His Asp Ser Leu Asp Val Asn Asn Pro Arg His Arg Glu Ile
145                 150                 155                 160
Ala Ala Phe Arg Leu Leu Ser Lys Met Pro Thr Met Ala Ala Met Cys
                165                 170                 175
Tyr Lys Tyr Ser Ile Gly Gln Pro Phe Val Tyr Pro Arg Asn Asp Leu
            180                 185                 190
Ser Tyr Ala Gly Asn Phe Leu Asn Met Met Phe Ser Thr Pro Cys Glu
        195                 200                 205
Pro Tyr Glu Val Asn Pro Ile Leu Glu Arg Ala Met Asp Arg Ile Leu
    210                 215                 220
Ile Leu His Ala Asp His Glu Gln Asn Ala Ser Thr Ser Thr Val Arg
225                 230                 235                 240
Thr Ala Gly Ser Ser Gly Ala Asn Pro Phe Ala Cys Ile Ala Ala Gly
                245                 250                 255
Ile Ala Ser Leu Trp Gly Pro Ala His Gly Gly Ala Asn Glu Ala Ala
            260                 265                 270
Leu Lys Met Leu Glu Glu Ile Ser Ser Val Lys His Ile Pro Glu Phe
        275                 280                 285
Phe Arg Arg Ala Lys Asp Lys Asn Asp Ser Phe Arg Leu Met Gly Phe
    290                 295                 300
Gly His Arg Val Tyr Lys Asn Tyr Asp Pro Arg Ala Thr Val Met Arg
305                 310                 315                 320
Glu Thr Cys His Glu Val Leu Lys Glu Leu Gly Thr Lys Asp Asp Leu
                325                 330                 335
Leu Glu Val Ala Met Glu Leu Glu Asn Ile Ala Leu Asn Asp Pro Tyr
            340                 345                 350
Phe Ile Glu Lys Lys Leu Tyr Pro Asn Val Asp Phe Tyr Ser Gly Ile
        355                 360                 365
Ile Leu Lys Ala Met Gly Ile Pro Ser Ser Met Phe Thr Val Ile Phe

370                 375                 380
Ala Met Ala Arg Thr Val Gly Trp Ile Ala His Trp Ser Glu Met His
385                 390                 395                 400
Ser Asp Gly Met Lys Ile Ala Arg Pro Arg Gln Leu Tyr Thr Gly Tyr
                405                 410                 415
Glu Lys Arg Asp Phe Lys Ser Asp Ile Lys Arg
            420                 425

<210> 10
<211> 447
<212> PRT
<213> Pantoea ananatis

<400> 10
Met Asp Gln Thr Tyr Ser Leu Glu Ser Phe Leu Asn His Val Gln Lys
1               5                   10                  15
Arg Asp Pro Asn Gln Thr Glu Phe Ala Gln Ala Val Arg Glu Val Met
            20                  25                  30
Thr Thr Leu Trp Pro Phe Leu Glu Gln Asn Pro Lys Tyr Arg Gln Met
        35                  40                  45
Ser Leu Leu Glu Arg Leu Val Glu Pro Glu Arg Val Ile Gln Phe Arg
    50                  55                  60
Val Val Trp Val Asp Asp Arg Asn Gln Ile Gln Val Asn Arg Ala Trp
65                  70                  75                  80
Arg Val Gln Phe Ser Ser Ala Ile Gly Pro Tyr Lys Gly Gly Met Arg
                85                  90                  95
Phe His Pro Ser Val Asn Leu Ser Ile Leu Lys Phe Leu Gly Phe Glu
            100                 105                 110
Gln Thr Phe Lys Asn Ala Leu Thr Thr Leu Pro Met Gly Gly Gly Lys
        115                 120                 125
Gly Gly Ser Asp Phe Asp Pro Lys Gly Lys Ser Glu Gly Glu Val Met
    130                 135                 140
Arg Phe Cys Gln Ala Leu Met Thr Glu Leu Tyr Arg His Leu Gly Ala
145                 150                 155                 160
Asp Thr Asp Val Pro Ala Gly Asp Ile Gly Val Gly Gly Arg Glu Val
                165                 170                 175
Gly Phe Met Ala Gly Met Met Lys Lys Leu Ser Asn Asn Thr Ala Cys
            180                 185                 190
Val Phe Thr Gly Lys Gly Leu Ser Phe Gly Gly Ser Leu Ile Arg Pro
        195                 200                 205
Glu Ala Thr Gly Tyr Gly Leu Val Tyr Phe Thr Glu Ala Met Leu Lys
    210                 215                 220

Arg His Gly Met Gly Phe Glu Gly Met Arg Val Ser Val Ser Gly Ser
225                 230                 235                 240
Gly Asn Val Ala Gln Tyr Ala Ile Glu Lys Ala Met Glu Phe Gly Ala
                245                 250                 255
Arg Val Ile Thr Ala Ser Asp Ser Ser Gly Thr Val Val Asp Glu Ser
            260                 265                 270
Gly Phe Thr Lys Glu Lys Leu Ala Arg Leu Ile Glu Ile Lys Ala Ser
        275                 280                 285
Arg Asp Gly Arg Val Ala Asp Tyr Ala Lys Glu Phe Gly Leu Val Tyr
    290                 295                 300
Leu Glu Gly Gln Gln Pro Trp Ser Leu Pro Val Asp Ile Ala Leu Pro
305                 310                 315                 320
Cys Ala Thr Gln Asn Glu Leu Asp Val Asp Ala Ala His Gln Leu Ile
                325                 330                 335
Ala Asn Gly Val Lys Ala Val Ala Glu Gly Ala Asn Met Pro Thr Thr
            340                 345                 350
Ile Glu Ala Thr Glu Leu Phe Gln Gln Ala Gly Val Leu Phe Ala Pro
        355                 360                 365
Gly Lys Ala Ala Asn Ala Gly Gly Val Ala Thr Ser Gly Leu Glu Met
    370                 375                 380
Pro Gln Asn Ala Ala Arg Leu Gly Trp Lys Ala Glu Lys Val Asp Ala
385                 390                 395                 400
Arg Leu His His Ile Met Leu Asp Ile His His Ala Cys Val Glu His
                405                 410                 415
Gly Gly Glu Gly Glu Gln Thr Asn Tyr Val Gln Gly Ala Asn Ile Ala
            420                 425                 430
Gly Phe Val Lys Val Ala Asp Ala Met Leu Ala Gln Gly Val Ile
        435                 440                 445

<210> 11
<211> 883
<212> PRT
<213> Pantoea ananatis

<400> 11
Met Asn Glu Gln Tyr Ser Ala Leu Arg Ser Asn Val Ser Met Leu Gly
1               5                   10                  15
Lys Val Leu Gly Glu Thr Ile Lys Asp Ala Leu Gly Glu His Ile Leu
            20                  25                  30
Glu Arg Val Glu Thr Ile Arg Lys Leu Ser Lys Ser Ser Arg Ala Gly
        35                  40                  45
Asn Asp Ala Asn Arg Gln Glu Leu Leu Thr Thr Leu Gln Asn Leu Ser

50                  55                  60
Asn Asp Glu Leu Leu Pro Val Ala Arg Ala Phe Ser Gln Phe Leu Asn
65                  70                  75                  80
Leu Ala Asn Thr Ala Glu Gln Tyr His Ser Ile Ser Pro Lys Gly Glu
                85                  90                  95
Ala Ala Ser Asn Pro Glu Val Ile Ala Arg Thr Leu Arg Lys Leu Lys
            100                 105                 110
Asn Gln Pro Glu Leu Ser Glu Asp Thr Ile Lys Lys Ala Val Glu Ser
        115                 120                 125
Leu Ser Leu Glu Leu Val Leu Thr Ala His Pro Thr Glu Ile Thr Arg
    130                 135                 140
Arg Thr Leu Ile His Lys Met Val Glu Val Asn Ala Cys Leu Lys Gln
145                 150                 155                 160
Leu Asp Asn Lys Asp Ile Ala Asp Tyr Glu His Asn Gln Leu Met Arg
                165                 170                 175
Arg Leu Arg Gln Leu Ile Ala Gln Ser Trp His Thr Asp Glu Ile Arg
            180                 185                 190
Lys Leu Arg Pro Ser Pro Val Asp Glu Ala Lys Trp Gly Phe Ala Val
        195                 200                 205
Val Glu Asn Ser Leu Trp Gln Gly Val Pro Asn Tyr Leu Arg Glu Leu
    210                 215                 220
Asn Glu Gln Leu Glu Glu Asn Leu Gly Tyr Lys Leu Pro Val Glu Phe
225                 230                 235                 240
Val Pro Val Arg Phe Thr Ser Trp Met Gly Gly Asp Arg Asp Gly Asn
                245                 250                 255
Pro Asn Val Thr Ala Asp Ile Thr Arg His Val Leu Leu Leu Ser Arg
            260                 265                 270
Trp Lys Ala Thr Asp Leu Phe Leu Lys Asp Ile Gln Val Leu Val Ser
        275                 280                 285
Glu Leu Ser Met Val Glu Ala Thr Pro Glu Leu Leu Ala Leu Val Gly
    290                 295                 300
Glu Glu Gly Ala Ala Glu Pro Tyr Arg Tyr Leu Met Lys Asn Leu Arg
305                 310                 315                 320
Ser Arg Leu Met Ala Thr Gln Ala Trp Leu Glu Ala Arg Leu Lys Gly
                325                 330                 335
Glu Glu Leu Pro Lys Pro Glu Gly Leu Leu Thr Gln Asn Glu Glu Leu
            340                 345                 350
Trp Glu Pro Leu Tyr Ala Cys Tyr Gln Ser Leu Gln Ala Cys Gly Met
        355                 360                 365
Gly Ile Ile Ala Asn Gly Asp Leu Leu Asp Thr Leu Arg Arg Val Lys
    370                 375                 380
Cys Phe Gly Val Pro Leu Val Arg Ile Asp Ile Arg Gln Glu Ser Thr

385                 390                 395                 400
Arg His Thr Glu Ala Leu Gly Glu Leu Thr Arg Tyr Leu Gly Ile Gly
                405                 410                 415
Asp Tyr Glu Ser Trp Ser Glu Ala Asp Lys Gln Ala Phe Leu Ile Arg
            420                 425                 430
Glu Leu Asn Ser Lys Arg Pro Leu Leu Pro Arg Asn Trp Gln Pro Ser
        435                 440                 445
Ala Glu Thr Arg Glu Val Leu Asp Thr Cys Gln Val Ile Ala Glu Ala
    450                 455                 460
Pro Gln Gly Ser Ile Ala Ala Tyr Val Ile Ser Met Ala Lys Thr Pro
465                 470                 475                 480
Ser Asp Val Leu Ala Val His Leu Leu Leu Lys Glu Ala Gly Ile Gly
                485                 490                 495
Phe Ala Met Pro Val Ala Pro Leu Phe Glu Thr Leu Asp Asp Leu Asn
            500                 505                 510
Asn Ala Asn Asp Val Met Thr Gln Leu Leu Asn Ile Asp Trp Tyr Arg
        515                 520                 525
Gly Leu Ile Gln Gly Lys Gln Met Val Met Ile Gly Tyr Ser Asp Ser
    530                 535                 540
Ala Lys Asp Ala Gly Val Met Ala Ala Ser Trp Ala Gln Tyr Gln Ala
545                 550                 555                 560
Gln Asp Ala Leu Ile Lys Thr Cys Glu Lys Ala Gly Ile Glu Leu Thr
                565                 570                 575
Leu Phe His Gly Arg Gly Gly Ser Ile Gly Arg Gly Gly Ala Pro Ala
            580                 585                 590
His Ala Ala Leu Leu Ser Gln Pro Pro Gly Ser Leu Lys Gly Gly Leu
        595                 600                 605
Arg Val Thr Glu Gln Gly Glu Met Ile Arg Phe Lys Tyr Gly Leu Pro
    610                 615                 620
Glu Ile Thr Val Ser Ser Leu Ser Leu Tyr Thr Gly Ala Ile Leu Glu
625                 630                 635                 640
Ala Asn Leu Leu Pro Pro Pro Glu Pro Lys Glu Ser Trp Arg Arg Ile
                645                 650                 655
Met Asp Glu Leu Ser Val Ile Ser Cys Asp Val Tyr Arg Gly Tyr Val
            660                 665                 670
Arg Glu Asn Lys Asp Phe Val Pro Tyr Phe Arg Ser Ala Thr Pro Glu
        675                 680                 685
Gln Glu Leu Gly Lys Leu Pro Leu Gly Ser Arg Pro Ala Lys Arg Arg
    690                 695                 700
Pro Thr Gly Gly Val Glu Ser Leu Arg Ala Ile Pro Trp Ile Phe Ala
705                 710                 715                 720
Trp Thr Gln Asn Arg Leu Met Leu Pro Ala Trp Leu Gly Ala Gly Thr

725                 730                 735
Ala Leu Gln Lys Val Val Glu Asp Gly Lys Gln Ser Glu Leu Glu Ala
            740                 745                 750
Met Cys Arg Asp Trp Pro Phe Phe Ser Thr Arg Leu Gly Met Leu Glu
        755                 760                 765
Met Val Phe Ala Lys Ala Asp Leu Trp Leu Ala Glu Tyr Tyr Asp Gln
    770                 775                 780
Arg Leu Val Asp Lys Ala Leu Trp Pro Leu Gly Lys Glu Leu Arg Asn
785                 790                 795                 800
Leu Gln Glu Glu Asp Ile Lys Val Val Leu Ala Ile Ala Asn Asp Ser
                805                 810                 815
His Leu Met Ala Asp Leu Pro Trp Ile Ala Glu Ser Ile Gln Leu Arg
            820                 825                 830
Asn Ile Tyr Thr Asp Pro Leu Asn Val Leu Gln Ala Glu Leu Leu His
        835                 840                 845
Arg Ser Arg Gln Ala Glu Lys Glu Gly Gln Glu Pro Asp Pro Arg Val
    850                 855                 860
Glu Gln Ala Leu Met Val Thr Ile Ala Gly Ile Ala Ala Gly Met Arg
865                 870                 875                 880
Asn Thr Gly

<210> 12
<211> 6038
<212> DNA
<213> Artificial Sequence

<220>
<223> Description of Artificial Sequence: plasmid pSTVCB

<220>
<221> CDS
<222> (2129)..(3439)

<400> 12
cgtatggcaa tgaaagacgg tgagctggtg atatgggata gtgttcaccc ttgttacacc 60
gttttccatg agcaaactga aacgttttca tcgctctgga gtgaatacca cgacgatttc 120
cggcagtttc tacacatata ttcgcaagat gtggcgtgtt acggtgaaaa cctggcctat 180
ttccctaaag ggtttattga gaatatgttt ttcgtctcag ccaatccctg ggtgagtttc 240
accagttttg atttaaacgt ggccaatatg gacaacttct tcgcccccgt tttcaccatg 300
ggcaaatatt atacgcaagg cgacaaggtg ctgatgccgc tggcgattca ggttcatcat 360
gccgtctgtg atggcttcca tgtcggcaga atgcttaatg aattacaaca gtactgcgat 420
gagtggcagg gcggggcgta atttttttaa ggcagttatt ggtgccctta aacgcctggt 480
gctacgcctg aataagtgat aataagcgga tgaatggcag aaattcgaaa gcaaattcga 540
cccggtcgtc ggttcagggc agggtcgtta aatagccgct tatgtctatt gctggtttac 600
cggtttattg actaccggaa gcagtgtgac cgtgtgcttc tcaaatgcct gaggccagtt 660
tgctcaggct ctccccgtgg aggtaataat tgacgatatg atcatttatt ctgcctccca 720
gagcctgata aaaacggtta gcgcttcgtt aatacagatg taggtgttcc acagggtagc 780
cagcagcatc ctgcgatgca gatccggaac ataatggtgc agggcgcttg tttcggcgtg 840
ggtatggtgg caggccccgt ggccggggga ctgttgggcg ctgccggcac ctgtcctacg 900
agttgcatga taaagaagac agtcataagt gcggcgacga tagtcatgcc ccgcgcccac 960
cggaaggagc taccggacag cggtgcggac tgttgtaact cagaataaga aatgaggccg 1020
ctcatggcgt tccaatacgc aaaccgcctc tccccgcgcg ttggccgatt cattaatgca 1080
gctggcacga caggtttccc gactggaaag cgggcagtga gcgcaacgca attaatgtga 1140
gttagctcac tcattaggca ccccaggctt tacactttat gcttccggct cgtatgttgt 1200
gtggaattgt gagcggataa caatttcaca caggaaacag ctatgaccat gattacgcca 1260
agcttgcatg cctgcaggtc gactctagag gatccccgtc gacaatagcc tgaatctgtt 1320
ctggtcgaac cttggaaggt ccgcagccga aacggccgtc gccagggatg aactcagagg 1380
gcagggtggg gaagtcggtc atgtcttcgg gcaactttct gcgcttggaa gtaaaagggc 1440
cagggatcgt taacgatctg acccaacaac tataaccctg aagctgtcag ttcctagcac 1500
cctagattct tcacgcagtc tcccaaacga tgaaaaacgc ccaaaactgg cgacaccgaa 1560
ctattgaaaa cgcggggatt agttgaccag ccaccaattt gggggtagct caaagttttg 1620
caaagttttc aatttctagg ttgttaatat cccctgaggt tgcgttatag ggtggcgaat 1680
tgcatgggga aagctactcg gcacccatcc ttgtcgcgtg catcacaaac tttgctaaac 1740
tgtgcaccag tccacttatt gtgggatttt taatgcctta aaggccagca tttttcaccc 1800
tctagcgggg ttgaatgctg gccttgaggg tgcagaacta aatagcagca catcggcaca 1860
attgatctga gttctattgg cgtgaccgtg gctactgatt acggtggctg tgggtggtcg 1920
ggaatgatgt aaccaacgtg attgtggggg aattggctct cacttcggat atggctaaac 1980
cgcatttatc ggtatagcgt gttaaccgga ccagattggg aaagaaatgt gtcgagtaac 2040
aaaaactgac atgcgcttgg cgcatcccag ttggtaagaa taaacgggac tacttccgta 2100
atccggaaga gtttttttcc gaacaaat atg ttt gaa agg gat atc gtg gct 2152
act gat aac aac aag gct gtc ctg cac tac ccc ggt ggc gag ttc gaa 2200
atg gac atc atc gag gct tct gag ggt aac aac ggt gtt gtc ctg ggc 2248
aag atg ctg tct gag act gga ctg atc act ttt gac cca ggt tat gtg 2296
agc act ggc tcc acc gag tcg aag atc acc tac atc gat ggc gat gcg 2344
gga atc ctg cgt tac cgc ggc tat gac atc gct gat ctg gct gag aat 2392
gcc acc ttc aac gag gtt tct tac cta ctt atc aac ggt gag cta cca 2440
acc cca gat gag ctt cac aag ttt aac gac gag att cgc cac cac acc 2488
ctt ctg gac gag gac ttc aag tcc cag ttc aac gtg ttc cca cgc gac 2536
gct cac cca atg gca acc ttg gct tcc tcg gtt aac att ttg tct acc 2584
tac tac cag gac cag ctg aac cca ctc gat gag gca cag ctt gat aag 2632
gca acc gtt cgc ctc atg gca aag gtt cca atg ctg gct gcg tac gca 2680
cac cgc gca cgc aag ggt gct cct tac atg tac cca gac aac tcc ctc 2728
aat gcg cgt gag aac ttc ctg cgc atg atg ttc ggt tac cca acc gag 2776
cca tac gag atc gac cca atc atg gtc aag gct ctg gac aag ctg ctc 2824
atc ctg cac gct gac cac gag cag aac tgc tcc acc tcc acc gtt cgt 2872
atg atc ggt tcc gca cag gcc aac atg ttt gtc tcc atc gct ggt ggc 2920
atc aac gct ctg tcc ggc cca ctg cac ggt ggc gca aac cag gct gtt 2968
ctg gag atg ctc gaa gac atc aag agc aac cac ggt ggc gac gca acc 3016
gag ttc atg aac aag gtc aag aac aag gaa gac ggc gtc cgc ctc atg 3064
ggc ttc gga cac cgc gtt tac aag aac tac gat cca cgt gca gca atc 3112
gtc aag gag acc gca cac gag atc ctc gag cac ctc ggt ggc gac gat 3160
ctt ctg gat ctg gca atc aag ctg gaa gaa att gca ctg gct gat gat 3208
tac ttc atc tcc cgc aag ctc tac ccg aac gta gac ttc tac acc ggc 3256
ctg atc tac cgc gca atg ggc ttc cca act gac ttc ttc acc gta ttg 3304
ttc gca atc ggt cgt ctg cca gga tgg atc gct cac tac cgc gag cag 3352
ctc ggt gca gca ggc aac aag atc aac cgc cca cgc cag gtc tac acc 3400
ggc aac gaa tcc cgc aag ttg gtt cct cgc gag gag cgc taaatttagc 3449
ggatgattct cgttcaactt cggccgaagc cacttcgtct gtcataatga cagggatggt 3509
ttcggccgtt tttgcatgaa accaaaaaat acgattttca aggagcatgt acagcacatg 3569
gaaaagccac agattgagct accggtcggt ccagcaccgg aagatctcgt aatctctgac 3629
atcatcgttg gcgaaggagc agaagcccgc ccaggtggag aagttgaggt ccactatgtg 3689
ggcgttgact ttgaaaccgg cgaggagttt gactcttcct gggatcgtgg acagaccagc 3749
cagttcccac tcaacggcct cattgcaggt tggcaagagg gaattccagg catgaaggtc 3809
ggcggacgtc gtcagctgac cattccgcca gaggctgctt acggccctga gggttccggc 3869
cacccactgt ctggccgtac cctggtgttc atcatcgatt tgatcagcgc ataattttct 3929
ttactgcgct aaacgctcaa atcgtgtgaa gcgactgtcg cgtcccgccc tctccggatt 3989
gttatccaat tcggagaggg cgttgctgat tgtgccgaga atttcttcaa caaagtgctc 4049
ggtttcggcg acgatcccgt cgataagccc ttggcttaaa agtgcgtgcg cctgcacgcc 4109
ttgtcgctct atgatttccg cggcgtggtt ggtgtcgcgg aagaggatgg ccgaggcgcc 4169
ctctggtggc aatgcggaca gccacgcgtt ttcggccgcg tagaccagat cggcgggcag 4229
catggccagc gcgccaccgc caacgccctg accaataatg accgaaacgg tggggagggg 4289
agcgtcgata agcttgcatg cctgcaggtc gactctagag gatccccggg taccgagctc 4349
gaattcactg gccgtcgttt tacaacgtcg tgactgggaa aaccctggcg ttacccaact 4409
taatcgcctt gcagcacatc cccctttcgc cagctggcgt aatagcgaag aggcccgcac 4469
cgatcgccct tcccaacagt tgcgcagcct gaatggcgaa tgagcttatc gatgataagc 4529
tgtcaaacat gagaattaca acttatatcg tatggggctg acttcaggtg ctacatttga 4589
agagataaat tgcactgaaa tctagaaata ttttatctga ttaataagat gatcttcttg 4649
agatcgtttt ggtctgcgcg taatctcttg ctctgaaaac gaaaaaaccg ccttgcaggg 4709
cggtttttcg aaggttctct gagctaccaa ctctttgaac cgaggtaact ggcttggagg 4769
agcgcagtca ccaaaacttg tcctttcagt ttagccttaa ccggcgcatg acttcaagac 4829
taactcctct aaatcaatta ccagtggctg ctgccagtgg tgcttttgca tgtctttccg 4889
ggttggactc aagacgatag ttaccggata aggcgcagcg gtcggactga acggggggtt 4949
cgtgcataca gtccagcttg gagcgaactg cctacccgga actgagtgtc aggcgtggaa 5009
tgagacaaac gcggccataa cagcggaatg acaccggtaa accgaaaggc aggaacagga 5069
gagcgcacga gggagccgcc aggggaaacg cctggtatct ttatagtcct gtcgggtttc 5129
gccaccactg atttgagcgt cagatttcgt gatgcttgtc aggggggcgg agcctatgga 5189
aaaacggctt tgccgcggcc ctctcacttc cctgttaagt atcttcctgg catcttccag 5249
gaaatctccg ccccgttcgt aagccatttc cgctcgccgc agtcgaacga ccgagcgtag 5309
cgagtcagtg agcgaggaag cggaatatat cctgtatcac atattctgct gacgcaccgg 5369
tgcagccttt tttctcctgc cacatgaagc acttcactga caccctcatc agtgccaaca 5429
tagtaagcca gtatacactc cgctagcgct gatgtccggc ggtgcttttg ccgttacgca 5489
ccaccccgtc agtagctgaa caggagggac agctgataga aacagaagcc actggagcac 5549
ctcaaaaaca ccatcataca ctaaatcagt aagttggcag catcacccga cgcactttgc 5609
gccgaataaa tacctgtgac ggaagatcac ttcgcagaat aaataaatcc tggtgtccct 5669
gttgataccg ggaagccctg ggccaacttt tggcgaaaat gagacgttga tcggcacgta 5729
agaggttcca actttcacca taatgaaata agatcactac cgggcgtatt ttttgagtta 5789
tcgagatttt caggagctaa ggaagctaaa atggagaaaa aaatcactgg atataccacc 5849
gttgatatat cccaatggca tcgtaaagaa cattttgagg catttcagtc agttgctcaa 5909
tgtacctata accagaccgt tcagctggat attacggcct ttttaaagac cgtaaagaaa 5969
aataagcaca agttttatcc ggcctttatt cacattcttg cccgcctgat gaatgctcat 6029
ccggaattt 6038

<210> 13
<211> 437
<212> PRT
<213> Brevibacterium lactofermentum

<400> 13

## Description

### BACKGROUND OF THE INVENTION

### Field of the Invention

The present invention relates to a method for producing L-glutamic acid by fermentation. L-Glutamic acid is widely used as a raw material of seasonings and so forth.

### Description of the Related Art

L-Glutamic acid is mainly produced by fermentation using an L-glutamic acid-producing bacterium of the so-called coryneform bacterium belonging to the genus *Brevibacterium, Corynebacterium* or *Microbacterium* or a mutant strain thereof. Moreover, methods utilizing a microorganism belonging to the genus *Bacillus, Streptomyces, Penicillium, Pseudomonas,Arthrobacter, Serratia, Candida, Serratia, Aerobacter aerogenes* (currently *Enterobacter aerogenes),* mutant strain of *Escherichia coli* or the like are known. Furthermore, the inventors of the present invention proposed a method of producing L-glutamic acid using a microorganism belonging to the genus *Klebsiella, Erwinia* or *Pantoea* (U.S. Patent No. 6,197,559) and a method of producing L-glutamic acid using an *Enterobacter* bacterium (U.S. Patent No. 6,331,419).

Furthermore, various techniques for improving L-glutamic acid-producing ability by enhancing activities of L-glutamic acid biosynthetic enzymes through use of recombinant DNA techniques have been disclosed. For example, it was reported that introduction of a gene encoding citrate synthase derived from *Escherichia coli* or *Corynebacterium glutamicum* was effective for enhancement of L-glutamic acid-producing ability in bacterium belonging to the genus *Corynebacterium* or *Brevibacterium* (Japanese Patent Publication (Japanese Patent Publication (Kokoku) No. 7-121228). In addition, Japanese Patent Laid-open (Kokai) No. 61-268185 discloses a cell harboring recombinant DNA containing a glutamate dehydrogenase gene derived from *Corynebacterium* bacteria. Further, Japanese Patent Laid-open No. 63-214189 discloses a technique for increasing L-glutamic acid-producing ability by amplifying genes encoding glutamate dehydrogenase, isocitrate dehydrogenase, aconitate hydratase and a citrate synthase.

L-glutamic acid productivity has been considerably increased by the aforementioned breeding of microorganisms or improvement of production methods. However, in order to respond to further increases in demand in the future, the development of methods which provide more efficient production of L-glutamic acid at a lower cost are still necessary, and therefore, still represent a need in the art.

On the other hand, a method of performing L-glutamic acid fermentation while precipitating L-glutamic acid which accumulates in culture broth has been developed (European Patent Application Laid-open No. 1078989). Because usual L-glutamic acid-producing bacteria cannot grow under acid conditions, the L-glutamic acid fermentation was conventionally performed under neutral conditions. Contrary to such conventional techniques, a microorganism having an ability to produce L-glutamic acid under acidic conditions was screened and it has been known that L-glutamic acid can be produced and accumulated in the medium while precipitating the L-glutamic acid by culturing the obtained microorganism *(Enterobacter agglomerans)* in a liquid medium in which pH is controlled so that L-glutamic acid is precipitated,.

Furthermore, a method for producing L-glutamic acid including culturing such an L-glutamic acid-producing bacterium that can grow under acidic conditions as described above in a medium in which total content of organic acids that inhibit growth of the bacterium is an amount that does not inhibit growth of the bacterium (European Patent Application Laid-open No. 1233070) and a method for producing L-glutamic acid comprising culturing such a bacterium as described above at a first pH optimal for growth of the microorganism and then culturing the bacterium at a second pH optimal for L-glutamic acid production by the microorganism and lower than the first pH (European Patent Application Laid-open No. 1233068) are known. Furthermore, a method for producing and accumulating L-glutamic acid in a medium while precipitating the L-glutamic acid in the medium, wherein an operation is performed to make crystals of L-glutamic acid exist in the medium during a period where L-glutaminc acid concentration in the medium is lower than the concentration at which natural crystallization of L-glutamic acid occurs (European Patent Application Laid-open No. 1233069) is known.

The crystals of L-glutamic acid exist in two forms, α- and β-form crystals (H. Takahashi, T. Takenishi, N. Nagashima, Bull. Chem. Soc. Japan, 35, 923 (1962), J. D. Bernal, Z. Krist., 78, 363 (1931); S. Hirokawa, Acta Cryst., 8, 637 (1955)). The β-form crystals are more stable in water, while the α-form crystals show better precipitating properties and are easy to handle in that, for example, the crystals can be effectively separated from crystallized slurry. In the method described in European Patent Application Laid-open No. 1233069, the α-form crystals can be selectively crystallized when the α-form crystals are used in operation of making the crystals of L-glutamic acid exist in the medium.

In addition, as a method for lowering the content of β-form crystals in L-glutamic acid crystals, methods of adding phenylalanine, leucine, tyrosine, cysteine, asparaginic acid, lysine, histidine, arginine, or alanine (Japanese Patent Publication (Kokoku) No. 36-17712, and Japanese Patent Publication (Kokoku) No. 38-16459) or adding ribonucleic acid, carboxy methyl cellulose, pectin, polyacrylic acid or salts thereof, or alginic acid or salts thereof (Japanese Patent Publication (Kokoku) No. 45-11286) have been disclosed. Those methods, however, have been examined at the isoelectric point (pH 3.2) of L-glutamic acid but no effect has been reported at slightly higher pH, approximately pH 4.5.

### Disclosure of the Invention

An object of the present invention is to provide an improved method for producing L-glutamic acid using a microorganism such as a bacterium belonging to the genus *Pantoea* having L-glutamic acid-producing ability.

The inventors of the present application found that super solubility of L-glutamic acid can be increased by making L-lysine exist in a medium in a method for producing L-glutamic acid by fermentation while precipitating L-glutamic acid to be accumulated in a culture medium, thereby α-form crystals of L-glutamic acid can be selectively crystallized in the medium when the concentration of L-lysine is at a certain level or more. Furthermore, when an operation of making crystals of L-glutamic acid exist in a medium is performed, the operation should be performed when the L-glutamic acid in the medium is equal to or higher than the saturation concentration and is lower than the concentration at which natural crystallization of L-glutamic acid occurs. However, the inventors of the present invention found that the timing of the operation can be broadened by increasing the super solubility of L-glutamic acid.

The present invention has been completed based on the above findings and the gist of the present invention is as follows.
(1) A method for producing L-glutamic acid by fermentation, which comprises culturing a microorganism that can metabolize a carbon source at a specific pH in a liquid medium containing L-glutamic acid at a saturation concentration and the carbon source and has an ability to accumulate L-glutamic acid in a liquid medium having said pH in an amount exceeding the amount corresponding to said saturation concentration of L-glutamic acid in a medium of which pH is controlled so that L-glutamic acid is precipitated to cause accumulation of L-glutamic acid in the medium while precipitating the L-glutamic acid,
   wherein the method comprises making L-lysine exist in the medium when L-glutaminc acid concentration in the medium is lower than the concentration at which natural crystallization of L-glutamic acid occurs so that α-form crystals of L-glutamic acid is precipitated.
(2) The method of (1), wherein the microorganism belongs to the genus *Pantoea.*
(3) The method of (2), wherein the microorganism is *Pantoea ananatis.*
(4) The method of any one of (1) to (3), wherein the amount of added L-lysine is 900 mg/L or more.
(5) The method of any one of (1) to (4), wherein pH of the medium at the time of making L-lysine exist is 3.0 to 5.0.
(6) The method of any one of (1) to (5), wherein an operation of making the α-form crystals of the L-glutamic acid exist in the medium is performed before occurrence of natural crystallization of L-glutamic acid.

### Brief Description of the Drawings

Fig. 1 shows a restriction map of a DNA fragment of pTWVEK101 derived from *Pantoea ananatis.*
Fig. 2 shows construction of a plasmid pMWCPG containing genes *gltA, ppc* and *gdhA.*
Fig. 3 shows construction of a plasmid RSF-Tet containing the replication origin of the wide host range plasmid RSF1010 and tetracycline resistence gene.
Fig. 4 shows construction of a plasmid RSFCPG containing the replication origin of the wide host range plasmid RSF1010, tetracycline resistence gene, *gltA* gene, *ppc* gene and *gdhA* gene.
Fig. 5 shows construction of a plasmid pSTVCB containing the *gltA* gene.
Fig. 6 is a graph showing a relationship between concentration and super solubility of L-lysine.
Fig. 7 is a graph showing a relationship between concentration of added L-lysine or L-phenylalanine and crystal form of precipitated L-glutamic acid.

### Best Mode for Carrying Out the Invention

Hereafter, the present invention will be explained in detail.

The present invention is a method for producing L-glutamic acid by fermentation, which comprises culturing a microorganism that can metabolize a carbon source at a specific pH in a liquid medium containing L-glutamic acid at a saturation concentration and the carbon source and has an ability to accumulate L-glutamic acid in a liquid medium having said pH in an amount exceeding the amount corresponding to said saturation concentration of L-glutamic acid (henceforth also referred to as an "L-glutamic acid-accumulating microorganism") in a medium of which pH is controlled so that L-glutamic acid is precipitated to cause accumulation of L-glutamic acid in the medium while precipitating the L-glutamic acid, wherein the method comprises making L-lysine exist in the medium when L-glutaminc acid concentration in the medium is lower than the concentration at which natural crystallization of L-glutamic acid occurs so that α-form crystals of L-glutamic acid is precipitated.

The above L-glutamic acid-accumulating microorganism can be obtained, for example, as follows. A sample containing microorganisms is inoculated into a liquid medium containing L-glutamic acid at a saturation concentration and a carbon source, at a specific pH, and a strain that metabolizes the carbon source is selected. Although the specific pH is not particularly limited, it is usually about 5.0 or less, preferably about 4.5 or less, further preferably about 4.3 or less. The L-glutamic acid-accumulating microorganism is used for production of L-glutamic acid by fermentation with precipitation of the L-glutamic acid accompanied. If the pH is too high, it becomes difficult to allow the microorganism to produce L-glutamic acid in an amount sufficient for precipitation. Therefore, pH is preferably in the aforementioned range.

If pH of an aqueous solution containing L-glutamic acid is lowered, the solubility of L-glutamic acid significantly falls around pKa of γ-carboxyl group (4.25, 25°C). The solubility becomes the lowest at the isoelectric point (pH 3.2) and L-glutamic acid exceeding the amount corresponding to the saturation concentration is precipitated. While it depends on the medium composition, L-glutamic acid is dissolved in an amount of 10-20 g/L at pH 3.2, 30-40 g/L at pH 4.0 and 50-60 g/L at pH 4.7, at about 30°C. Usually pH does not need to be made 3.0 or lower, because the L-glutamic acid precipitating effect reaches its upper limit when pH goes below a certain value. However, pH may be 3.0 or less.

In addition, the expression that a microorganism "can metabolize a carbon source" means that the microorganism can proliferate or can consume a carbon source even though it cannot proliferate, that is, it indicates that the microorganism catabolizes a carbon source such as sugars or organic acids. Specifically, for example, if a microorganism proliferates when it is cultured in a liquid medium containing L-glutamic acid at a saturation concentration at pH 5.0 to 4.0, preferably pH 4.5 to 4.0, more preferably pH 4.3 to 4.0, most preferably about pH 4.0, at an appropriate temperature, for example, 28°C, 37°C or 50°C, for 2 to 4 days, this microorganism is a microorganism that can metabolize the carbon source in the medium. Further, for example, if a microorganism consume a carbon source even though the microorganism does not proliferate, when it is cultured in a synthetic liquid medium containing L-glutamic acid at a saturation concentration at pH 5.0 to 4.0, preferably pH 4.5 to 4.0, more preferably pH 4.3 to 4.0, most preferably about pH 4.0, at an appropriate temperature, for example, 28°C, 37°C or 50°C, for 2 to 4 days, the microorganism is a microorganism that can metabolize the carbon source in the medium.

The microorganism that can metabolize a carbon source include a microorganism that can grow in the aforementioned liquid medium. The expression that a microorganism "can grow" means that it can proliferate or can produce L-glutamic acid even though it cannot proliferate. Specifically, for example, if a microorganism proliferates when it is cultured in a liquid medium containing L-glutamic acid at a saturation concentration at pH 5.0 to 4.0, preferably pH 4.5 to 4.0, more preferably pH 4.3 to 4.0, most preferably about pH 4.0, at an appropriate temperature, for example, 28°C, 37°C or 50°C, for 2 to 4 days, this microorganism is a microorganism that can grow in the medium. Further, for example, if a microorganism increases an amount of L-glutamic acid in a synthetic liquid medium even though the microorganism does not proliferate, when the microorganism is cultured in the synthetic liquid medium containing L-glutamic acid at a saturation concentration at pH 5.0 to 4.0, preferably pH 4.5 to 4.0, more preferably pH 4.3 to 4.0, most preferably about pH 4.0, at an appropriate temperature, for example, 28°C, 37°C or 50°C, for 2 to 4 days, this microorganism is a microorganism that can grow in the medium.

The selection of a microorganism as described above may be repeated two or more times under the same conditions or with changing pH or the concentration of L-glutamic acid. A selection for an early stage can be performed in a medium containing L-glutamic acid at a concentration lower than the saturation concentration, and thereafter a subsequent selection can be performed in a medium containing L-glutamic acid at a saturation concentration. Further, strains with favorable properties such as superior proliferation rate may be selected.

The L-glutamic acid-accumulating microorganism has an ability to accumulate L-glutamic acid in an amount exceeding the amount corresponding to the saturation concentration of L-glutamic acid in a liquid medium, in addition to the properties described above. The pH of the aforementioned liquid medium is preferably the same as or close to that of the medium used for screening a microorganism having the aforementioned properties. Usually, a microorganism becomes sensitive to L-glutamic acid at a high concentration as pH becomes lower. Therefore, it is preferred that pH is not low in view of resistance to L-glutamic acid, but low pH is preferred in view of production of L-glutamic acid with its precipitation accompanied. To satisfy these conditions, pH can be in the range of 3 to 5, preferably 4 to 5, more preferably 4 to 4.7, further preferably 4 to 4.5, particularly preferably 4.0 to 4.3.

Examples of the L-glutamic acid-accumulating microorganism or breeding materials therefore include, but are not limited to, microorganisms belonging to the genus *Pantoea, Enterobacter, Klebsiella,* Serratia, *Erwinia, Escherichia, Corynebacterium, Brevibacterium, Alicyclobacillus, Bacillus, Saccharomyces* or the like. Among these, microorganisms belonging to the genus *Pantoea* are preferred. Hereafter, the microorganism of the present invention will be explained mainly for microorganisms belonging to the genus *Pantoea.* However, the microorganism is not limited to those belonging to the genus *Pantoea,* and those belonging to other genera can be similarly used.

An example of a microorganism belonging to the *Pantoea* includes, but is not limited to, *Pantoea ananatis,* preferably the *Pantoea ananatis* AJ13355 strain. This strain was isolated from soil in Iwata-shi, Shizuoka, Japan as a strain that can proliferate in a medium containing L-glutamic acid and a carbon source at low pH.

The *Pantoea ananatis* AJ13355 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 and received an accession number of FERM P-16644. It was then converted to an international deposit under the provisions of Budapest Treaty on January 11, 1999 and received an accession number of FERM BP-6614.
The above strain was identified as *Enterobacter agglomerans* when it was isolated and deposited as the *Enterobacter agglomerans* AJ13355 strain. However, it was recently re-classified as *Pantoea ananatis* on the basis of nucleotide sequencing of 16S rRNA and so forth (see the examples section).

Although the strains AJ13356 and AJ13601 that had been derived from AJ13355 strain were also deposited at the aforementioned depository as *Enterobacter agglomerans,* they are described as *Pantoea ananatis* in this specification.

The L-glutamic acid-accumulating microorganism may be a microorganism originally having L-glutamic acid-producing ability or one having L-glutamic acid-producing ability imparted or increased by breeding through mutagenesis, recombinant DNA techniques or the like.

The L-glutamic acid-producing ability can be imparted or increased by, for example, increasing an activity of an enzyme that catalyzes a biosynthetic reaction of L-glutamic acid. The L-glutamic acid-producing ability can also be increased by decreasing or eliminating activity of an enzyme that catalyzes a reaction which branches off from the biosynthetic pathway of L-glutamic acid and generates a compound other than L-glutamic acid.

An example of the enzyme that catalyzes the biosynthetic reaction of L-glutamic acid includes, but are not limited to, glutamate dehydrogenase (hereafter, also referred to as "GDH"), glutamine synthetase, glutamate synthase, isocitrate dehydrogenase, aconitate hydratase, citrate synthase (hereafter, also referred to as "CS"), phosphoenolpyruvate carboxylase (hereafter, also referred to as "PEPC"), pyruvate dehydrogenase, pyruvate kinase, enolase, phosphoglyceromutase, phosphoglycerate kinase, glyceraldehyde-3-phosphate dehydrogenase, triosephosphate isomerase, fructose bisphosphate aldolase, phosphofructokinase, glucose phosphate isomerase and so forth. Among these enzymes, one, two or three of CS, PEPC and GDH are preferred. Furthermore, it is preferred that the activities of all the three enzymes, CS, PEPC and GDH, are enhanced in the L-glutamic acid-accumulating microorganism. In particular, CS from *Brevibacterium lactofermentum* is preferred, because it is not subject to inhibition by α-ketoglutaric acid, L-glutamic acid and NADH.

In order to enhance the activity of CS, PEPC or GDH, for example, a gene encoding CS, PEPC or GDH can be cloned on an appropriate plasmid and a host microorganism can be transformed with the obtained plasmid. The copy number of the gene encoding CS, PEPC or GDH (hereafter, abbreviated as "*gltA* gene", "*ppc* gene" and "*gdhA* gene", respectively) in the cell of the transformant increases, resulting in the increase of the activity of CS, PEPC or GDH.

The cloned *gltA, ppc* and *gdhA* genes are introduced into the aforementioned starting parent strain solely or in combination of arbitrary two or three kinds of them. When two or three kinds of the genes are introduced, two or three kinds of the genes may be cloned on one kind of plasmid and introduced into the host, or separately cloned on two or three kinds of plasmids that can coexist and introduced into the host.

Two or more kinds of genes encoding an enzyme of the same kind, but derived from different microorganisms, may be introduced into the same host.

The plasmids described above are not particularly limited so long as they are autonomously replicable in a cell of a microorganism belonging to, for example, the genus *Pantoea* or the like. Exmaples of the plasmids includes pUC19, pUC18, pBR322, pHSG299, pHSG298, pHSG399, pHSG398, RSF1010, pMW119, pMW118, pMW219, pMW218, pACYC177, pACYC184 and so forth. Vectors of phage DNA can also be used for introducing the aforementioned genes.

Transformation can be performed by, for example, the method of D.M. Morrison (Methods in Enzymology, 68, 326 (1979)), the method wherein permeability of DNA of recipient bacterium cells is increased by treating the cells with calcium chloride (Mandel M. and Higa A., J. Mol. Biol., 53, 159 (1970)), electroporation (Miller J.H., "A Short Course in Bacterial Genetics", Cold Spring Harbor Laboratory Press, U.S.A., 1992) or the like.

The activity of CS, PEPC or GDH can also be increased by allowing multiple copies of the *gltA* gene, *the ppc* gene or the *gdhA* gene to be present on chromosomal DNA of the aforementioned starting parent strain. In order to introduce the *gltA* gene, *the ppc* gene or the *gdhA* gene into chromosomal DNA of a bacterium belonging to the genus *Pantoea* in multiple copy number, the sequence present on chromosomal DNA in multiple copy number such as a repetitive DNA and inverted repeats present at the end of a transposable element can be used. Alternatively, multiple copies can be introduced into chromosomal DNA by incorporating them into a transposon and transferring it. As a result, the copy number of *gltA* gene, the *ppc* gene or the *gdhA* gene in cells of a transformant strain is increased, and thus the activity of CS, PEPC or GDH is increased.

As organisms used as a source of the *gltA* gene, *the ppc* gene or the *gdhA* gene of which copy number is to be increased, any organism can be used so long as it has activity of CS, PEPC or GDH. Examples of the organism preferably include, but are not limited to, bacteria, which are procaryote, belonging to the genus *Pantoea, Enterobacter, Klebsiella, Erwinia, Serratia, Escherichia, Corynebacterium, Brevibacterium* or *Bacillus.* Specifically, *Escherichia* coli, *Brevibacterium lactofermentum* and so forth are encompassed by the present invention. The *gltA* gene, the *ppc* gene and the *gdhA* gene can be obtained from chromosomal DNA of the microorganisms described above.

The *gltA* gene, the *ppc* gene and the *gdhA* gene can be obtained using a mutant strain which is deficient in the activity of CS, PEPC or GDH to isolate a DNA fragment that supplements its auxotrophy from chromosomal DNA of the aforementioned microorganism. Furthermore, since the nucleotide sequences of these genes of bacteria belonging to the genera *Escherichia* and *Corynebacterium* are known (Biochemistry, 22, pp.5243-5249, (1983); J. Biochem., 95, pp.909-916, (1984); Gene, 27, pp.193-199, (1984); Microbiology, 140, pp.1817-1828, (1994); Mol. Gen. Genet., 218, pp.330-339, (1989); Molecular Microbiology, 6, pp.317-326, (1992)), they can also be obtained by PCR utilizing primers synthesized based on each nucleotide sequence and chromosomal DNA as a template. It is known that, in enterobacteria such as bacteria belonging to the genus *Enterobacter* or *Klebsiella,* introduction of a *gltA* gene from a coryneform bacterium is more effective on enhancement of L-glutamic acid-producing ability compared with that of a *gltA* gene from a bacterium of the same species (European Patent Application Laid-open No. 0999282). In this patent document, the strains of *Pantoea ananatis* described in this specification are described as *Enterobacter agglomerans.*

The activity of CS, PEPC or GDH can also be increased by enhancing the expression of the *gltA* gene, the *ppc* gene or the *gdhA* gene, besides the aforementioned amplification of the genes. For example, the expression can be enhanced by replacing a promoter for the *gltA* gene, the *ppc* gene or the *gdhA* gene with another stronger promoter. For example, *lac* promoter, *trp* promoter, *trc* promoter, *tac* promoter, P_{R} promoter and P_{L} promoter of the lamda phage and so forth are known as strong promoters. The *gltA* gene, the *ppc* gene and the *gdhA* gene of which promoter is replaced are cloned on a plasmid and introduced into the host microorganism, or introduced into the chromosomal DNA of the host microorganism using repetitive DNA, inverted repeat, transposon or the like.

The activity of CS, PEPC or GDH can also be increased by replacing the promoter of the *gltA* gene, the *ppc* gene or the *gdhA* gene on the chromosome with another stronger promoter (see WO87/03006 and Japanese Patent Application Laid-open No. 61-268183), or inserting a strong promoter in the upstream of the coding sequence of each gene (see Gene, 29, pp.231-241 (1984)). Specifically, homologous recombination can be performed between the *gltA* gene, the *ppc* gene or the *gdhA* gene of which promoter is replaced with a stronger one or DNA containing a part thereof and the corresponding gene on the chromosome.

Examples of the enzyme that catalyzes the reaction which branches off from the biosynthetic pathway of the L-glutamic acid and generates a compound other than L-glutamic acid include α-ketoglutarate dehydrogenase (hereafter, also referred to as "αKGDH"), isocitrate lyase, phosphate acetyltransferase, acetate kinase, acetohydroxy acid synthase, acetolactate synthase, formate acetyltransferase, lactate dehydrogenase, glutamate decarboxylase, 1-pyrroline dehydrogenase and so forth. Among these enzymes, αKGDH is preferred.

In order to decrease or eliminate the activities of the aforementioned enzymes in a microorganism belonging to the genus *Pantoea* or the like, mutations for decreasing or eliminating the intracellular activity of the enzymes can be introduced into genes of the aforementioned enzymes by a usual mutagenesis treatment method or a genetic engineering method.

Examples of the mutagenesis treatment method include, for example, methods utilizing irradiation with X-ray or ultraviolet ray, methods utilizing treatment with a mutagenesis agent such as N-methyl-N'-nitro-N-nitrosoguanidine, and so forth. The site of a gene where the mutation is introduced may be in a coding region coding for an enzyme protein or a region for regulating expression such as a promoter.

Examples of the genetic engineering methods include, for example, methods utilizing gene recombination, transduction, cell fusion and so forth. For example, a drug resistance gene is inserted into a cloned target gene to prepare a gene that has lost its function (defective gene). Subsequently, this defective gene is introduced into a cell of a host microorganism, and the target gene on the chromosome is replaced with the aforementioned defective gene by utilizing homologous recombination (gene disruption).

Decrease or deficiency of intracellular activity of the target enzyme in a cell and the degree of decrease of the activity can be confirmed by measuring the enzyme activity of a cell extract or a purified fraction thereof obtained from a candidate strain and comparing it with that of a wild-type strain. For example, the αKGDH activity can be measured by the method of Reed et al. (Reed L.J. and Mukherjee B.B., Methods in Enzymology, 13, pp.55-61 (1969)).

Depending on the target enzyme, a target mutant strain can be selected based on a phenotype of the mutant strain. For example, a mutant strain wherein the αKGDH activity is eliminated or decreased cannot proliferate or shows a markedly reduced proliferation rate in a minimal medium containing glucose or a minimal medium containing acetic acid or L-glutamic acid as an exclusive carbon source under aerobic culture conditions. However, normal proliferation is enabled even under the same condition by adding succinic acid or lysine, methionine and diaminopimelic acid to a minimal medium containing glucose. By utilizing these phenomena as indicators, a mutant strain with decreased αKGDH activity or deficient in the activity can be selected.

A method for preparing an αKGDH gene-deficient strain of *Brevibacterium lactofermentum* by utilizing homologous recombination is described in detail in WO95/34672. Similar methods can be applied to other microorganisms.

Furthermore, techniques such as cloning of genes and digestion and ligation of DNA, transformation and so forth are described in detail in Molecular Cloning, 2nd Edition, Cold Spring Harbor Press (1989) and so forth.

Specific example of a mutant strain deficient in αKGDH activity or with decreased αKGDH activity obtained as described above includes *Pantoea ananatis* AJ13356. *Pantoea ananatis* AJ13356 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 and received an accession number of FERM P-16645. It was then converted to an international deposit under the provisions of Budapest Treaty on January 11, 1999 and received an accession number of FERM BP-6615. The *Pantoea ananatis* AJ13356 is deficient in αKGDH activity as a result of disruption of the aKGDH-E1 subunit gene *(sucA).* This strain was identified as *Enterobacter agglomerans* when it was isolated and deposited as the *Enterobacter agglomerans* AJ13356 strain. However, it was recently re-classified as *Pantoea ananatis* on the basis of nucleotide sequencing of 16S rRNA and so forth (see the examples section).

When *Pantoea ananatis,* which is an example of the microorganism used in the present invention, is cultured in a medium containing a saccharide, mucus is extracellularly secreted, occasionally resulting in low operation efficiency. Therefore, when *Pantoea ananatis* having such a property of secreting mucus is used, it is preferable to use a mutant strain that secretes less mucus compared with a wild-type strain. Examples of mutagenesis treatment include, for example, methods utilizing irradiation with X-ray or ultraviolet ray, method utilizing treatment with a mutagenesis agent such as N-methyl-N'-nitro-N-nitrosoguanidine, and so forth. A mutant strain with decreased secretion of mucus can be selected by inoculating mutagenized bacterial cells in a medium containing a saccharide, for example, LB medium plate containing 5 g/L of glucose, culturing them with tilting the plate about 45 degrees and selecting a colony that does not show flowing down of mucus.

In the present invention, impartation or enhancement of L-glutamic acid-producing ability and impartation of other favorable properties such as mutation for less mucus secretion described above can be carried out in an arbitrary order.

As a gene used for the breeding of such L-glutamic acid-producing bacteria as described above, the nucleotide sequence of the *sucA* gene *of Pantoea ananatis* and the amino acid sequence of the aKGDH-E1 subunit encoded by the gene are shown SEQ ID NO: 1 and SEQ ID NO: 3.

Furthermore, the nucleotide sequence of the plasmid RSFCPG containing the *gltA* gene, *gdhA* gene and *ppc* gene derived from *Escherichia coli* (see Reference Example 1) is shown in SEQ ID NO: 8. In SEQ ID NO: 8, the coding regions of the *gltA* gene, *gdhA* gene and *ppc* gene correspond to the nucleotide numbers 1770 to 487 (encoded by the complementary strand), 2598 to 3941 and 7869 to 5218 (encoded by the complementary strand), respectively. The amino acid sequences of CS, GDH and PEPC encoded by these genes are shown in SEQ ID NOS: 9, 10 and 11. Furthermore, the nucleotide sequence of plasmid pSTVCB containing the *gltA* gene derived from *Brevibacterium lactofermentum* (see Reference Example 1) and the amino acid sequence of CS encoded by this gene are shown in SEQ ID NO: 12 and SEQ ID NO: 13.

CS, GDH and PEPC may be, besides those of wild-type, those which have an amino acid sequence including substitution, deletion, insertion, addition or inversion of one or several amino acid residues that does not substantially degrade the activities of the enzymes. Although the number of "several" amino acid residues referred to herein differs depending on positions in the three-dimensional structures of the proteins or types of amino acid residues, it may be specifically between 2 to 30, preferably between 2 to 20, more preferably between 2 to 10.

Examples of DNA coding for the substantially same protein or peptide as CS, GDH or PEPC include DNA hybridizable with an open reading frame (ORF) of the nucleotide sequence shown in SEQ ID NO: 12 or 8 or a probe that can be prepared from the nucleotide sequence under stringent conditions and encoding a protein having the activity of CS, GDH or PEPC. The "stringent conditions" referred to herein include conditions under which so-called specific hybrid is formed, and non-specific hybrid is not formed. It is difficult to clearly express this condition by using any numerical value. However, for example, the stringent conditions include conditions under which DNAs having high homology, for example, DNAs having homology of not less than 50%, preferably not less than 70%, more preferably not less than 90%, most preferably not less than 95% hybridize with each other, but DNAs having homology lower than the above does not hybridize with each other. Alternatively, the stringent conditions include conditions whereby DNAs hybridize with each other at a salt concentration corresponding to an ordinary condition of washing in Southern hybridization, i.e., 1 x SSC, 0.1% SDS, preferably 0.1 x SSC, 0.1% SDS, at 60°C.

ORF of the nucleotide sequence of SEQ ID NO: 12 or SEQ ID NO: 8 or a partial sequence thereof can also be used as the probe. Such a probe can be prepared by PCR using oligonucleotides produced on the basis of the nucleotide sequence of SEQ ID NO: 8 or 12 as primers and a DNA fragment containing the nucleotide sequence of SEQ ID NO: 8 or 12 or a partial nucleotide sequence thereof as a template. When a DNA fragment having a length of about 300 bp is used as the probe, the washing conditions for the hybridization can be, for example, 2 x SSC and 0.1% SDS at 50°C.

It is sufficient that the deletion-type *sucA* gene used for gene disruption has homology to such a degree that it causes homologous recombination with the *sucA* gene on a chromosomal DNA of an objective microorganism. Such homology is preferably not less than 85%, more preferably not less than 90%, particularly preferably not less than 95%. Moreover, DNAs hybridizable under stringent conditions may cause homologous recombination.

Specific examples of such a strain obtained as described above include the AJ13601 strain derived from the aforementioned *Pantoea ananatis* AJ13355 strain. This strain was obtained by selection of a low mucus-producing strain from the AJ13355 strain, disruption of the aKGDH gene, introduction of the *gltA, ppc* and *gdhA* genes derived from *Escherichia coli,* and the *gltA* gene derived from *Brevibacterium lactofermentum,* selection of a strain showing resistance to L-glutamic acid of high concentration at a low pH, and selection of a strain showing superior growth ability and L-glutamic acid-producing ability.

By culturing the L-glutamic acid-accumulating microorganism in a liquid medium that is adjusted to pH conditions that allow precipitation of L-glutamic acid, L-glutamic acid can be produced and accumulated with its precipitation in the medium accompanied. The "conditions that allow precipitation of L-glutamic acid produced by the microorganism" referred to herein means conditions that allow precipitation of L-glutamic acid when the L-glutamic acid-accumulating microorganism produces and accumulates L-glutamic acid. Although pH of these conditions may vary depending on the L-glutamic acid-producing ability of the microorganism, it is usually 3 to 5, preferably 4.5 or less, more preferably 4 or less when the microorganism is a bacterium belonging to the genus *Pantoea.*

Furthermore, as for the aforementioned pH conditions that allow precipitation of L-glutamic acid, the pH is determined on conditions that the L-glutamic acid-accumulating microorganism can metabolize a carbon source in a liquid medium containing L-glutamic acid at a saturation concentration and the carbon source and exhibit an ability to accumulate L-glutamic acid in the medium in an amount exceeding the amount corresponding to the saturation concentration of L-glutamic acid in the medium at that pH.

When L-glutamic acid-accumulating microorganism is cultured in the medium under the above conditions to produce and accumulate L-glutamic acid while allowing precipitation of L-glutamic acid in the medium, the a-form crystals can be selectively precipitated by making a certain amount or more of L-lysine exist in the medium when the concentration of L-glutamic acid in the medium is lower than the concentration at which natural crystallization occurs. In the case where the L-glutamic acid-accumulating microorganism is cultured in the absence of L-lysine in the medium to produce and accumulate L-glutamic acid in the medium while precipitating L-glutamic acid in the medium, the a-form crystals of L-glutamic acid should exist as seed crystals in the medium in order to surely precipitate the a-form crystals. On the other hand, with the method of the present invention, the a-form crystals can be precipitated by making a certain amount or more of L-lysine exist in a medium even without the addition of seed crystals.

Method for making L-lysine exist in a medium includes, for example, a method of adding L-lysine to the medium. The addition of L-lysine is performed when the concentration of L-glutamic acid in the medium is lower than the concentration at which natural crystallization occurs. This is because the a-form crystals are hardly precipitated even if L-lysine is added to the medium after the occurrence of natural crystallization of β-form crystals. Preferably, L-lysine is added to the medium after allowing the concentration of L-glutamic acid in the medium to be equal to or higher than the saturation concentration. In addition, if the concentration of L-glutamic acid in the medium becomes less than the saturation concentration, L-lysine may be added to the medium. Furthermore, L-lysine may be accumulated in the medium when the concentration of L-glutamic acid in the medium is lower than the concentration at which natural crystallization thereof occurs by using a microorganism that accumulates L-lysine together with L-glutamic acid in a medium (for example, see WO96/06180). . The expression "natural crystallization" described above means that the accumulation of L-glutamic acid by a microorganism having an L-glutamic acid-producing ability leads to natural precipitation of L-glutamic acid in a medium as the L-glutamic acid concentration in the medium exceeds the saturation concentration.

In the present invention, the amount of L-lysine made to exist in a medium is 500 mg/L or more, typically 600 mg/L to 1500 mg/L, preferably 1000 mg/L or more, more preferably 1500 mg/L or more. In particular, when no seed crystal is added, the amount of L-lysine is 900 mg/L or more, preferably 1000 mg/L or more, more preferably 1500 mg/L or more.

The pH of a medium at the time of precipitating crystals of L-glutamic acid in the medium may be generally equal to pH for accumulating L-glutamic acid in the medium, but is preferably set to 3.0 to 5.0, more preferably 3.0 to 4.9, particularly preferably 4.5 to 4.9.

To the present invention, known methods of producing L-glutamic acid using L-glutamic acid-accumulating microorganism while precipitating L-glutamic acid can be applied, except that L-lysine is made exist in the medium when L-glutaminc acid concentration in the medium is lower than the concentration at which natural crystallization of L-glutamic acid occurs (for example, Japanese Patent Laid-open No. 2001-333769 (European Patent Application Laid-open No. 1078989), Japanese Patent Laid-open No. 2002-238591 (European Patent Application Laid-open No. 1233070), Japanese Patent Laid-open No. 2002-238592 (European Patent Application Laid-open No. 1233068), Japanese Patent Laid-Open No. 2002-238593 (European Patent Application Laid-open No. 1233069)).

For example, one of preferred embodiments of the method of the present invention is a method for producing L-glutamic acid which includes culturing an L-glutamic acid-accumulating microorganism in a medium having pH of 5.0 or less in which total content of organic acids that inhibit growth of the microorganism is an amount that does not inhibit growth of the microorganism, in which L-lysine is made exist in the medium when L-glutaminc acid concentration in the medium is lower than the concentration at which natural crystallization of L-glutamic acid occurs (see Japanese Patent Laid-open No. 2002-238591 (European Patent Application Laid-open No. 1233070)). In this embodiment, the organic acid that inhibits growth of a microorganism at pH of medium means an organic acid that has an inhibitory effect on growth of the microorganism when it exists at a certain concentration (usually 0.5 g/L or more) in the medium at the pH, and it is usually an organic acid having carbon number of 1 to 3, i.e., formic acid, acetic acid or propionic acid.

The total content of the organic acid is preferably 0.4 g/L or less, more preferably 0.3 g/L or less, further preferably 0.2 g/L or less.

Another preferred embodiment of the method of the present invention is a method for producing L-glutamic acid which includes culturing an L-glutamic acid-accumulating microorganism at a first pH optimal for growth of the microorganism and then culturing the microorganism at a second pH optimal for production of L-glutamic acid by the microorganism and lower than the first pH, and in which L-lysine is made exist in the medium when L-glutaminc acid concentration in the medium is lower than the concentration at which natural crystallization of L-glutamic acid occurs (see Japanese Patent Laid-open No. 2002-238592 (European Patent Application Laid-open No. 1233068)).

Another preferred embodiment of the method of the present invention is a method for producing L-glutamic acid which includes culturing an L-glutamic acid-accumulating microorganism at a first pH at which growth of the microorganism is not inhibited by organic acids contained in the medium and then culturing the microorganism at a second pH optimal for production of L-glutamic acid by the microorganism and lower than the first pH bacterium, and in which L-lysine is made exist in the medium when L-glutaminc acid concentration in the medium is lower than the concentration at which natural crystallization of L-glutamic acid occurs (see Japanese Patent Laid-open No. 2002-238591 (European Patent Application Laid-open No. 1233070)).

It was found that an L-glutamic acid-producing bacterium generally suffered from inhibition of growth by an organic acid under an acid condition, whereas it could consume an organic acid under a neutral condition (EP 1233070 A). Based on this property, by effecting cell growth at a neutral pH and then changing pH into acidic pH to produce L-glutamic acid, it becomes possible to obtain higher productivity and it also becomes possible to use various materials as a sugar source.

In this embodiment, the organic acid means an organic acid that has an inhibitory effect on growth of microorganism when it exists at a certain concentration (usually 0.5 g/L or more) in a medium at the second pH, and it is usually an organic acid having carbon number of 1 to 3, i.e., formic acid, acetic acid or propionic acid.

The first pH and the second pH are selected so that they meet the properties of an L-glutamic acid-producing bacterium to be used. These pH values can be easily measured by those skilled in the art. For example, the pH at which inhibition of growth of microorganism is not caused by an organic acid in a medium can be determined by culturing an L-glutamic acid-producing bacterium in media containing an organic acid adjusted to various pH values, measuring cell amounts based on absorbance or the like, and comparing the cell amounts with cell amounts of the L-glutamic acid-producing bacterium cultured under the same conditions except that the media do not contain the organic acid. The pH suitable for the production of L-glutamic acid refers to pH at which L-glutamic acid is accumulated in a medium, determinable by culturing an L-glutamic acid-producing bacterium in media of various pH values. Specifically, it can be determined by measuring amounts of L-glutamic acid accumulated in media of various pH values and comparing them.

The first pH is not particularly limited so long as growth of microorganism is not inhibited by the organic acid in the medium, but it is usually 5.0 to 8.0.

The second pH is preferably a pH at which the produced L-glutamic acid precipitates, and such pH is usually 3.0 to 5.0. The reduction of productivity by accumulation of L-glutamic acid at a high concentration can be obviated by performing the culture at the pH at which the produced L-glutamic acid precipitates.

The first pH and the second pH may not be strictly constant during the culture so long as the advantage of the present invention can be obtained, and they may fluctuate.

The L-glutamic acid-producing bacterium produces L-glutamic acid even at the first pH, and therefore pH is lowered by the produced L-glutamic acid. Therefore, the culture at the first pH is preferably performed while maintaining pH of the medium at the first pH by adding an alkalizing substance to the medium.

Although the alkalizing substance is not particularly limited so long as it does not adversely affect the growth of the L-glutamic acid-producing bacterium or L-glutamic acid production, ammonia gas is preferred.

The pH of the medium may be lowered from the first pH to the second pH by adding an acidic substance. However, pH is lowered by L-glutamic acid produced by the L-glutamic acid-producing bacterium during the culture as described above. Therefore, it is preferable to lower the pH of the medium from the first pH to the second pH by controlling the addition amount of the alkalizing substance, because the addition of the acidic substance can be omitted.

The culture at the first pH may be continued until the organic acid in the medium is depleted. The depletion means that the amount of the organic acid decreases to a level at which growth of the L-glutamic acid-producing bacterium is not inhibited during the culture at the second pH. Such a level of the organic acid can be easily measured by those skilled in the art. For example, the level can be determined by culturing an L-glutamic acid-producing bacterium in media containing an organic acid at various concentrations at the second pH, measuring cell amounts of the L-glutamic acid-producing bacterium, and comparing the cell amounts with cell amounts of the L-glutamic acid-producing bacterium cultured under the same conditions except that the media do not contain the organic acid. Generally, as the second pH becomes lower, the level of the organic acid also becomes lower.

A further preferred embodiment of the method of the present invention is a method for producing L-glutamic acid by fermentation including culturing an L-glutamic acid-accumulating bacterium in a medium of which pH is controlled so that L-glutamic acid produced by the microorganism is precipitated to cause accumulation of L-glutamic acid in the medium while precipitating the L-glutamic acid, wherein L-lysine is made exist in the medium when L-glutaminc acid concentration in the medium is lower than the concentration at which natural crystallization of L-glutamic acid occurs and an operation is performed to make a-form crystals of L-glutamic acid (seed crystals) exist in the medium before natural crystallization of L-glutamic acid occurs (see Japanese Patent Laid-open No. 2002-238593 (European Patent Application Laid-open No. 1233069)).

The operation of making seed crystals exist in the medium is performed before the occurrence of natural crystallization of β-form crystals of L-glutamic acid. In addition, seed crystals may be dissolved if the seed crystals are added before the L-glutamic acid concentration in a medium reaches the saturation concentration. Therefore, the seed crystals are preferably added after the L-glutamic acid concentration in the medium exceeds the saturation concentration but before the occurrence of natural crystallization of L-glutamic acid. In the present invention, making L-lysine exist in a medium to increase the L-glutamic acid concentration at which natural crystallization thereof occurs allows the timing of adding seed crystals to be broadened.

The operation to make a-form crystals of L-glutamic acid exist in the medium means an operation for artificially providing existence of crystals of L-glutamic acid in the medium. Examples of the operation include addition of a-form crystals, an operation of dissolving a certain amount of L-glutamic acid in the medium at the start of the culture and decreasing pH during the culture to forcibly precipitate crystals and so forth. Amount of a-form crystals made to exist in the medium is usually 0.01 to 10 g/L. The amount of L-glutamic acid crystals that exist in the medium and the concentration of L-glutamic acid can be measured by methods well known to those skilled in the art. Crystals of L-glutamic acid are measured after the medium is left standing, and the crystals are collected by decantation. The concentration of L-glutamic acid in the medium is a concentration of dissolved L-glutamic acid. When crystals precipitate in the medium, the concentration of L-glutamic acid means a value of L-glutamine acid concentration measured for a clear solution obtained by separating solid content from the medium by centrifugation (or filtration).

The operation for making the a-form crystals of L-glutamic acid exist in the medium is preferably addition of a-form crystals crystals of L-glutamic acid.

The preferred amount of crystals to be added is usually 0.2 g/L or more. If the crystals are added in the aforementioned amount or a larger amount, a-form crystals can be obtained with good reproducibility. Crystals of a-form can be handled more easily compared with crystals of β-form in view of the morphology thereof.

As the medium used for the present invention, a usual nutrient medium containing a carbon source, nitrogen source and inorganic salts as well as organic trace amount nutrients such as amino acids and vitamins as required can be used except that pH is adjusted to satisfy the predetermined conditions. Either a synthetic medium or natural medium may be used. Any carbon source and nitrogen source that can be used by a strain to be cultured may be used in the medium.

Saccharides such as glucose, glycerol, fructose, sucrose, maltose, mannose, galactose, starch hydrolysate and molasses are used as the carbon source. In addition, organic acids such as acetic acid and citric acid may be used each alone or in combination with another carbon source.

Ammonia, ammonium salts such as ammonium sulfate, ammonium carbonate, ammonium chloride, ammonium phosphate and ammonium acetate, nitrates and so forth are used as the nitrogen source.

Amino acids, vitamins, fatty acids, nucleic acids, those containing these substances such as peptone, casamino acid, yeast extract and soybean protein decomposition products are used as the organic trace nutrients. When an auxotrophic mutant strain that requires an amino acid and so forth for metabolization or growth is used, the required nutrient must be supplemented.

Phosphates, magnesium salts, calcium salts, iron salts, manganese salts and so forth are used as the inorganic salts.

As for the culture method, aeration culture at 20 to 42°C is usually performed provided that pH is controlled to be a predetermined value, preferably 3 to 5.

After completion of the culture, L-glutamic acid precipitated in the culture can be collected by centrifugation, filtration or the like. L-Glutamic acid dissolved in the medium can be also collected by known methods. For example, the L-glutamic acid can be isolated by concentrating the culture broth to crystallize it or isolated by ion exchange chromatography or the like. It is also possible to crystallize L-glutamic acid dissolved in the medium and then collect the crystallized L-glutamic acid together with the L-glutamic acid precipitated during the culture.

In an embodiment where L-glutamic acid exceeding a saturation concentration precipitates, the concentration of L-glutamic acid dissolved in the medium is maintained at a constant level. Therefore, influence of L-glutamic acid at a high concentration on microorganisms can be reduced. Accordingly, it also becomes possible to breed a microorganism having further improved L-glutamic acid-producing ability. Further, since L-glutamic acid is precipitated as crystals, acidification of the culture broth by accumulation of L-glutamic acid is suppressed, and therefore the amount of alkali used for maintaining pH of the culture broth can significantly be reduced.

### Examples

Hereafter, the present invention will be more specifically explained with reference to the following examples.

### Reference Example 1

<1> Screening of microorganism having L-glutamic acid resistance in acidic environment
   Screening of a microorganism having L-glutamic acid resistance in an acidic environment was performed as follows. One (1) g each of about 500 samples obtained from nature including soil, fruits, plant bodies, river water and so forth was suspended in 5 mL of sterilized water, and 200 µL thereof was applied on 20 mL of solid medium adjusted to pH 4.0 with HCl. The composition of the medium was as follows: 3 g/L of glucose, 1 g/L of ammonium sulfate, 0.2 g/L of magnesium sulfate heptahydrate, 0.5 g/L of potassium dihydrogenphosphate, 0.2 g/L of sodium chloride, 0.1 g/L of calcium chloride dihydrate, 0.01 g/L of ferrous sulfate heptahydrate, 0.01 g/L of manganese sulfate tetrahydrate, 0.72 mg/L of zinc sulfate dihydrate, 0.64 mg/L of copper sulfate pentahydrate, 0.72 mg/L of cobalt chloride hexahydrate, 0.4 mg/L of boric acid, 1.2 mg/L of sodium molybdate dihydrate, 50 *µ*g/L of biotin, 50 *µ*g/L of calcium pantothenate, 50 *µ*g/L of folic acid, 50 *µ*g/L of inositol, 50 *µ*g/L of niacin, 50 *µ*g/L of p-aminobenzoic acid, 50 *µ*g/L of pyridoxine hydrochloride, 50 *µ*g/L of riboflavin, 50 *µ*g/L of thiamin hydrochloride, 50 mg/L of cycloheximide and 20 g/L of agar.
   The media plated with the above samples were incubated at 28°C, 37°C or 50°C for 2 to 4 days and 378 strains forming colonies were obtained.
   Subsequently, each of the strains obtained as described above was inoculated in a test tube of 16.5 cm in length and 14 mm in diameter containing 3 mL of liquid medium (adjusted to pH 4.0 with HCl) containing a saturation concentration of L-glutamic acid and cultured at 28°C, 37°C or 50°C for 24 hours to 3 days with shaking. Then, the grown strains were selected. The composition of the aforementioned medium was follows: 40 g/L of glucose, 20 g/L of ammonium sulfate, 0.5 g/L of magnesium sulfate heptahydrate, 2 g/L of potassium dihydrogenphosphate, 0.5 g/L of sodium chloride, 0.25 g/L of calcium chloride dihydrate, 0.02 g/L of ferrous sulfate heptahydrate, 0.02 g/L of manganese sulfate tetrahydrate, 0.72 mg/L of zinc sulfate dihydrate, 0.64 mg/L of copper sulfate pentahydrate, 0.72 mg/L of cobalt chloride hexahydrate, 0.4 mg/L of boric acid, 1.2 mg/L of sodium molybdate dihydrate and 2 g/L of yeast extract.
   Thus, 78 strains of microorganisms showing L-glutamic acid resistance in an acidic environment were successfully obtained.
<2> Selection of strains showing superior growth rate from microorganisms having L-glutamic acid resistance in acidic environment
   The various microorganisms having L-glutamic acid resistance in an acidic environment obtained as described above are each inoculated into a test tube of 16.5 cm in length and 14 mm in diameter containing 3 mL of medium (adjusted to pH 4.0 with HCl) obtained by adding 20 g/L of glutamic acid and 2 g/L of glucose to M9 medium (Sambrook, J., Fritsh, E.F. and Maniatis, T., "Molecular Cloning", Cold Spring Harbor Laboratory Press, U.S.A., 1989), and the turbidity of the medium was measured in the time course to select strains showing a favorable growth rate. As a result, as a strain showing favorable growth, the AJ13355 strain was obtained from soil in Iwata-shi, Shizuoka, Japan. This strain was determined as *Enterobacter agglomerans* based on its bacteriological properties described above. *Enterobacter agglomerans* includes those re-classified into *Pantoea agglomerans, Pantoea ananatis, Pantoea stewartii* and so forth on the basis of nucleotide sequence analysis of 16S rRNA or the like, and the AJ13355 strain is classified into *Pantoea ananatis* among these.
<3> Acquisition of strain with less mucus secretion from *Pantoea ananatis* AJ13355 strain
   Since the *Pantoea ananatis* AJ13355 strain extracellularly secretes mucus when cultured in a medium containing a saccharide, operation efficiency is not favorable. Therefore, a strain with less mucus secretion was obtained by the ultraviolet irradiation method (Miller, J.H. et al., "A Short Course in Bacterial Genetics; Laboratory Manual", p.150, 1992, Cold Spring Harbor Laboratory Press, U.S.A.).
   The *Pantoea ananatis* AJ13355 strain was irradiated with ultraviolet ray for 2 minutes at a position 60 cm away from a 60-W ultraviolet lamp and cultured in LB medium overnight to fix mutation. The mutagenized strain was diluted and inoculated in LB medium containing 5 g/L of glucose and 20 g/L of agar so that about 100 colonies per plate emerge and cultured at 30°C overnight with tilting the plate about 45 degrees, and then 20 colonies showing not flowing down of mucus were selected.
   As a strain which satisfied conditions that no revertant emerged even after 5 times of subculture in LB medium containing 5 g/L of glucose and 20 g/L of agar, and which showed growth equivalent to the parent strain in LB medium, LB medium containing 5 g/L of glucose and M9 medium (Sambrook, J. et al., Molecular Cloning, 2nd Edition, Cold Spring Harbor Press, U.S.A., 1989) supplemented with 20 g/L of L-glutamic acid and 2 g/L of glucose and adjusted to pH 4.5 with HCl, SC17 strain was selected from the strains selected above.
<4> Construction of glutamic acid-producing bacterium from *Pantoea ananatis* SC17 strain
   (1) Preparation of aKGDH deficient strain from *Pantoea ananatis* SC17 strain
      A strain that was deficient in aKGDH and had enhanced L-glutamic acid biosynthetic system was prepared from the *Pantoea ananatis* SC17 strain.
      (i) Cloning of aKGDH gene (hereafter, referred to as *"sucAB")* of *Pantoea ananatis* AJ13355 strain
         The *sucAB* gene of the *Pantoea ananatis* AJ13355 strain was cloned by selecting a DNA fragment complementing the acetic acid-unassimilating property of the aKGDH-E1 subunit gene (hereafter, referred to as "sucA")-deficient strain of *Escherichia coli* from chromosomal DNA of the *Pantoea ananatis* AJ13355 strain.
         The chromosomal DNA of the *Pantoea ananatis* AJ13355 strain was isolated by a method usually employed for extracting chromosomal DNA from *Escherichia coli* (Text for Bioengineering Experiments, Edited by the Society for Bioscience and Bioengineering, Japan, pp.97-98, Baifukan, 1992). The pTWV228 (resistant to ampicillin), which is a commercial product of Takara Shuzo Co., Ltd, was used as a vector.
         The chromosomal DNA of the AJ13355 strain digested with *Eco*T221 and pTWV228 digested with *Pst*I were ligated using T4 ligase and the ligation mixture was used to transform the *sucA*-deficient *Escherichia coli* JRG465 strain (Herbert, J. et al., Mol. Gen. Genetics, 105, 182 (1969)). A strain growing in an acetate minimal medium was selected from the transformant strains obtained above, and a plasmid was extracted from the obtained strain and designated as pTWVEK101. The *Escherichia coli* JRG465 strain harboring pTWVEK101 recovered auxotrophy for succinic acid or L-lysine and L-methionine besides the trait of acetic acid-unassimilating property. This suggests that pTWVEK101 contained the *sucA* gene of *Pantoea ananatis.*

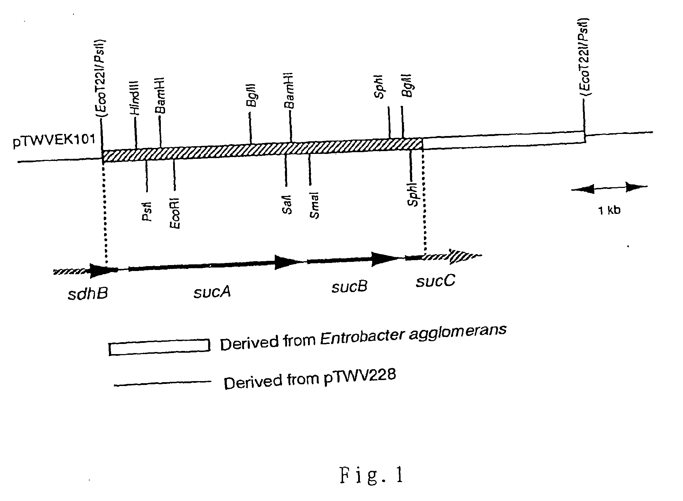

         Fig. 1 shows a restriction enzyme map of a DNA fragment derived from *Pantoea ananatis* in pTWVEK101. SEQ ID NO: 1 shows a result of sequencing the hatched portion in Fig. 1. In the nucleotide sequence, nucleotide sequences considered to be two full length ORFs and two nucleotide sequences considered to be partial sequences of ORFs were found. Each of SEQ ID NO: 2 to 5 shows the amino acid sequences which may be encoded by these ORFs or partial sequences thereof from 5' end in order. As a result of homology search for these, it was revealed that the portions of which nucleotide sequences were determined contained a 3' end partial sequence of the succinate dehydrogenase iron-sulfur protein gene *(sdhB),* full length *sucA* and aKGDH-E2 subunit gene *(sucB* gene), and a 5' end partial sequence of the succinyl CoA synthetase β subunit gene (*sucC* gene). When comparing the amino acid sequences deduced from these nucleotide sequences with those derived from *Escherichia coli* (Eur. J. Biochem., 141, pp.351-359 (1984); Eur. J. Biochem., 141, pp.361-374 (1984); Biochemistry, 24, pp.6245-6252 (1985)), each of the amino acid sequences showed very high homology to each other. In addition, it was found that a cluster of *sdhB-sucA-sucB-sucC* was constituted on the chromosome of *Pantoea ananatis* as in *Escherichia coli* (Eur. J. Biochem., 141, pp.351-359 (1984); Eur. J. Biochem., 141, pp.361-374 (1984); Biochemistry, 24, pp.6245-6252 (1985)).
      (ii) Acquisition of aKGDH-deficient strain derived from *Pantoea ananatis* SC17 strain
         The homologous recombination was performed using the *sucAB* gene of *Pantoea ananatis* obtained as described above to obtain an aKGDH-deficient strain of *Pantoea ananatis.*
         After pTWVEK101 was digested with *SphI* to excise a fragment containing *sucA,* the fragment was blunt-ended with Klenow fragment (Takara Shuzo Co., Ltd.) and ligated with pBR322 digested with *EcoRI* and blunt-ended with Klenow fragment, by using T4 DNA ligase (Takara Shuzo Co., Ltd.). The obtained plasmid was digested at the restriction enzyme *BglII* recognition site positioned approximately at the center of *suca* by using the enzyme, blunt-ended with Klenow fragment, and then ligated again by using T4 DNA ligase. It was considered that the *sucA* gene became unfunctional because a frameshift mutation was introduced into *sucA* of the plasmid newly constructed through the above procedure.
         The plasmid constructed as described above was digested with a restriction enzyme *Apa*LI*,* and subjected to agarose gel electrophoresis to recover a DNA fragment containing *sucA* into which the frameshift mutation was introduced and a tetracycline resistance gene derived from pBR322. The recovered DNA fragment was ligated again by using T4 DNA ligase to construct a plasmid for disrupting the aKGDH gene.
         The plasmid for disrupting the aKGDH gene obtained as described above was used to transform the *Pantoea ananatis* SC17 strain by electroporation (Miller, J.H., "A Short Course in Bacterial Genetics; Handbook", p.279, Cold Spring Harbor Laboratory Press, U.S.A., 1992), and a strain wherein *sucA* on the chromosome was replaced with a mutant *sucA* of the plasmid by homologous recombination was obtained by using the tetracycline resistance as a marker. The obtained strain was designated as SC17sucA strain.
         In order to confirm that the SC17sucA strain was deficient in the aKGDH activity, the enzyme activity was measured by the method of Reed et al. (Reed, L.J. and Mukherjee, B.B., Methods in Enzymology, 13, pp.55-61, (1969)) by using cells of the strain cultured in LB medium to the logarithmic growth phase. As a result, aKGDH activity of 0.073 (ΔABS/min/mg protein) was detected from the SC17 strain, whereas no aKGDH activity was detected from the SC17sucA strain, and thus it was confirmed that the *sucA* was eliminated as intended.
   (2) Enhancement of L-glutamic acid biosynthesis system *of Pantoea ananatis* SC17sucA strain

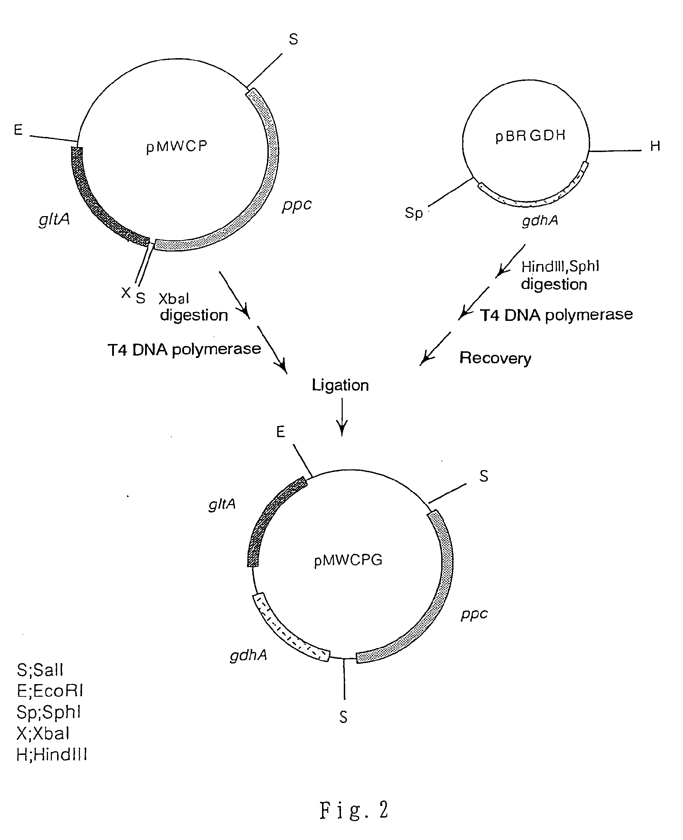

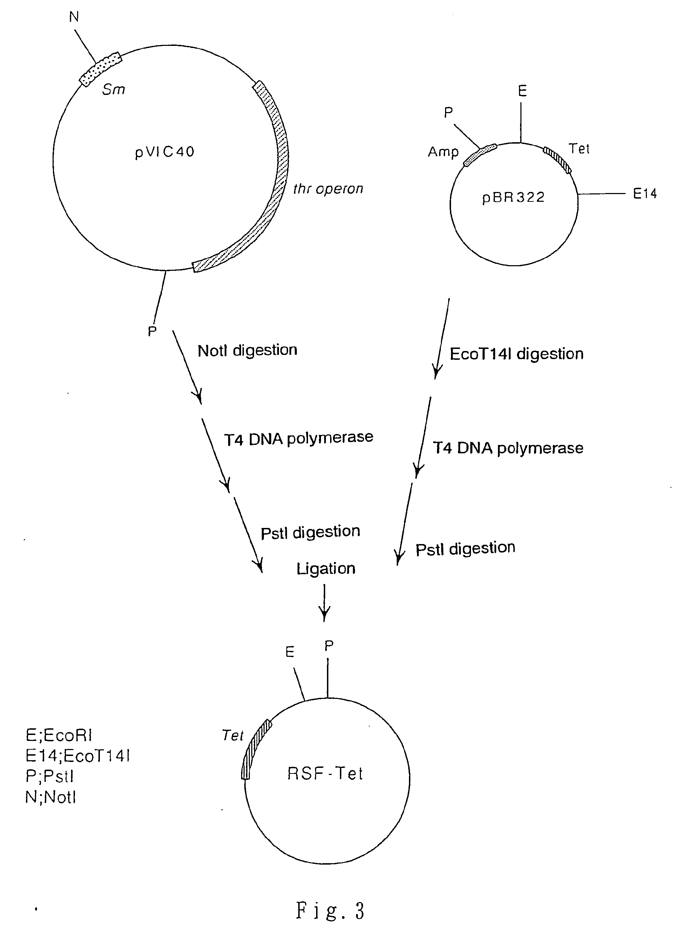

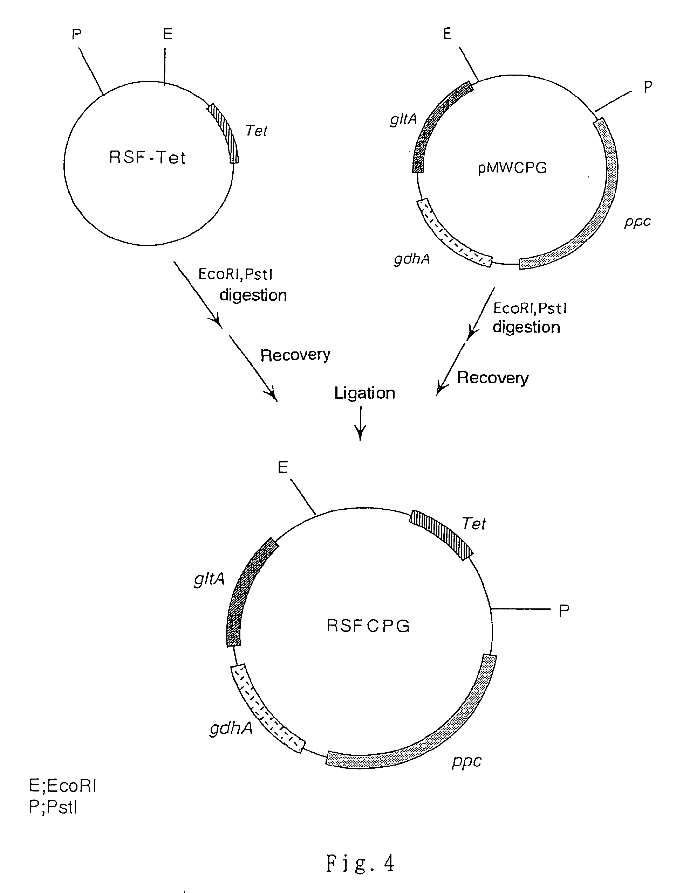

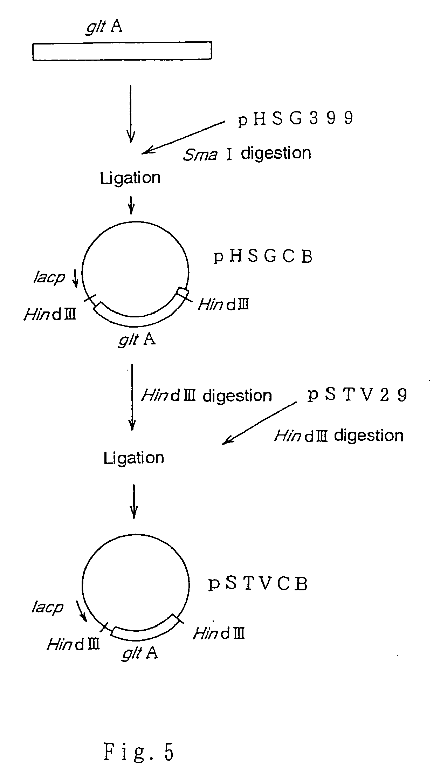

   Subsequently, the citrate synthase gene, phosphoenolpyruvate carboxylase gene and glutamate dehydrogenase gene derived from *Escherichia coli* were introduced into the SC17sucA strain.
   (i) Preparation of plasmid having *gltA* gene, *ppc* gene and *gdhA* gene derived from *Escherichia coli*
      The procedures of preparing a plasmid having the *gltA* gene, the *ppc* gene and the *gdhA* gene will be explained by referring to Figs. 2 and 3.
      A plasmid having the *gdhA* gene derived from *Escherichia coli,* pBRGDH (Japanese Patent Application Laid-open No. 7-203980), was digested with *Hin*dIII and *Sph*I, the both ends were blunt-ended by the T4 DNA polymerase treatment, and then the DNA fragment having the *gdhA* gene was purified and recovered. Separately, a plasmid having the *gltA* gene and *ppc* gene derived from *Escherichia coli,* pMWCP (W097/08294), was digested with *Xba*I, and then the both ends were blunt-ended by using T4 DNA polymerase. This was mixed with the above purified DNA fragment having the *gdhA* gene and ligated by using T4 ligase to obtain a plasmid pMWCPG, which corresponded to pMWCP further containing the *gdhA* gene (Fig. 2).
      Concurrently, the plasmid pVIC40 (Japanese Patent Application Laid-open No. 8-047397) having the replication origin of the wide-host-range plasmid RSF1010 was digested with *Not*I, treated with T4 DNA polymerase and digested with *Pst*I. pBR322 was digested with *Eco*T14I, treated with T4 DNA polymerase and digested with *Pst*I. The both products were mixed and ligated by using T4 ligase to obtain a plasmid RSF-Tet having the replication origin of RSF1010 and the tetracycline resistance gene (Fig. 3).
      Subsequently, pMWCPG was digested with *Eco*RI and *Pst*I*,* and a DNA fragment having the *gltA* gene, *the ppc* gene and the *gdh4* gene was purified and recovered. RSF-Tet was similarly digested with *Eco*RI and *Pst*I*,* and a DNA fragment having the replication origin of RSF1010 was purified and recovered. The both products were mixed and ligated by using T4 ligase to obtain a plasmid RSFCPG, which corresponded to RSF-Tet containing the *gltA* gene, *the ppc* gene and the *gdhA* gene (Fig. 4). It was confirmed that the obtained plasmid RSFCPG expressed the *gltA* gene, the *ppc* gene and the *gdhA* gene based on the supplementation of the auxotrophy of the *gltA* gene*-, ppc* gene- or *gdhA* gene- deficient strain derived from *Escherichia coli* and measurement of each enzyme activity.
   (ii) Preparation of plasmid having *gltA* gene derived from *Brevibacterium lactofermentum*
      A plasmid having the *gltA* gene derived from *Brevibacterium lactofermentum* was constructed as follows. PCR was performed by using the primer DNAs, having the nucleotide sequences shown in SEQ ID NOS: 6 and 7, which were prepared based on the nucleotide sequence of the *Corynebacterium glutamicum gltA* gene (Microbiology, 140, pp.1817-1828 (1994)), and chromosomal DNA of *Brevibacterium lactofermentum* ATCC13869 as a template to obtain a *gltA* gene fragment of about 3 kb. This fragment was inserted into a plasmid pHSG399 (purchased from Takara Shuzo Co., Ltd.) digested with *Sma*I to obtain a plasmid pHSGCB (Fig. 5). Subsequently, pHSGCB was digested with HindIII, and the excised *gltA* gene fragment of about 3 kb was inserted into a plasmid pSTV29 (purchased from Takara Shuzo Co., Ltd.) digested with *Hind*III to obtain a plasmid pSTVCB (Fig. 5). It was confirmed that the obtained plasmid pSTVCB expressed the *gltA* gene by measuring the enzyme activity in the *Pantoea ananatis* AJ13355 strain.
   (iii) Introduction of RSFCPG and pSTVCB into SC17sucA strain

   The *Pantoea ananatis* SC17sucA strain was transformed with RSFCPG by electroporation to obtain a transformant SC17sucA/RSFCPG strain showing tetracycline resistance. Further, the SC17sucA/RSFCPG strain was transformed with pSTVCB by electroporation to obtain a transformant SC17sucA/RSFCPG+pSTVCB strain showing chloramphenicol resistance.
<5> Acquisition of strain with improved resistance to L-glutamic acid in low pH environment

A strain with improved resistance to L-glutamic acid at a high concentration in a low pH environment (hereafter, also referred to as "strain with high-concentration Glu-resistance at low pH") was isolated from the *Pantoea ananatis* SC17sucA/RSFCPG+pSTVCB strain.

The SC17sucA/RSFCPG+pSTVCB strain was cultured overnight at 30°C in LBG medium (10 g/L of trypton, 5 g/L of yeast extract, 10 g/L of NaCl, 5 g/L of glucose), and the cells washed with saline was appropriately diluted and plated on an M9-E medium (4 g/L of glucose, 17 g/L of Na₂HPO₄ •12H₂O, 3 g/L of KH₂PO₄, 0.5 g/L of NaCl, 1 g/L of NH₄Cl, 10 mM of MgSO₄, 10 *µ*M of CaCl₂, 50 mg/L of L-lysine, 50 mg/L of L-methionine, 50 mg/L of DL-diaminopimelic acid, 25 mg/L of tetracycline, 25 mg/L of chloramphenicol, 30 g/L of L-glutamic acid, adjusted to pH 4.5 with aqueous ammonia) plate. A colony emerged after culture at 32°C for 2 days was obtained as a strain with high-concentration Glu-resistance at low pH.

For the obtained strain, growth level in M9-E liquid medium was measured and L-glutamic acid-producing ability was tested in a 50-ml volume large test tube containing 5 ml of L-glutamic acid production test medium (40 g/L of glucose, 20 g/L of ammonium sulfate, 0.5 g/L of magnesium sulfate heptahydrate, 2 g/L of potassium dihydrogenphosphate, 0.5 g/L of sodium chloride, 0.25 g/L of calcium chloride dihydrate, 0.02 g/L of ferrous sulfate heptahydrate, 0.02 g/L of manganese sulfate tetrahydrate, 0.72 mg/L of zinc sulfate dihydrate, 0.64 mg/L of copper sulfate pentahydrate, 0.72 mg/L of cobalt chloride hexahydrate, 0.4 mg/L of boric acid, 1.2 mg/L of sodium molybdate dihydrate, 2 g/L of yeast extract, 200 mg/L of L-lysine hydrochloride, 200 mg/L of L-methionine, 200 mg/L of DL-a,e-diaminopimelic acid, 25 mg/L of tetracycline hydrochloride and 25 mg/L of chloramphenicol). A strain that exhibited the best growth level and the same L-glutamic acid-producing ability as that of its parent strain, the SC17/RSFCPG+pSTVCB strain, was designated as *Pantoea ananatis* AJ13601. The AJ13601 strain was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on August 18, 1999 and received an accession number of FERM P-17516. It was then converted to an international deposit under the provisions of Budapest Treaty on July 6, 2000 and received an accession number of FERM BP-7207.

### Example 1: Studies on effect of L-lysine on maximum saturated concentration of L-glutamice acid

Cells of the *Pantoea ananatis* AJ13601 strain cultured at 30°C for 14 hours in the LBG agar medium (10 g/L of trypton, 5 g/L of yeast extract, 10 g/L of NaCl, 15 g/L of agar) containing 25 mg/L of tetracycline hydrochloride and 25 mg/L of chloramphenicol were scraped from one plate and inoculated to 300 ml of seed culture medium having the following composition and contained in a 1 L-volume jar fermenter, and seed culture was performed under conditions of 34°C and pH 6.0.

**[Composition of seed culture medium]**

| | |
|---|---|
| Sucrose | 50 g/L |
| MgSO₄·7H₂O | 0.4 g/L |
| KH₂PO₄ | 2.0 g/L |
| Yeast extract | 4.0 g/L |
| FeSO₄ · 7H₂O | 0.01 g/L |
| MnSO₄ · 5H₂O | 0.01 g/L |
| L-Lysine hydrochloride | 0.4 g/L |
| DL-Methionine | 0.4 g/L |
| e-Diaminopimelic acid | 0.4 g/L |
| Tetracycline hydrochloride | 25 mg/L |
| Chloramphenicol | 25 mg/L |

pH was adjusted to 6.0 by adding ammonia gas during the culture. The seed culture was finished by observing depletion of the saccharide in the medium as an index, and the seed culture medium corresponding to 20% volume of the main culture medium was inoculated to 300 ml of the main culture medium contained in a 1 L-volume jar fermenter to perform the main culture under conditions of 34°C and pH 4.5. The composition of the main culture medium is shown below.

**[Composition of main culture medium]**

| | |
|---|---|
| Glucose | 50 g/L |
| (NH₄)₂SO₄ | 5.0 g/L |
| MgSO₄·7H₂O | 0.4 g/L |
| KH₂PO₄ | 6.0 g/L |
| NaCl | 1.5 g/L |
| FeSO₄·7H₂O | 0.01 g/L |
| MnSO₄·5H₂O | 0.01 g/L |
| L-Lysine hydrochloride | 0.8 g/L |
| DL-Methionine | 0.6 g/L |
| DL-a,e-Diaminopimelic acid | 0.6 g/L |
| Tetracycline hydrochloride | 25 mg/L |
| Chloramphenicol | 25 mg/L |
| Calcium chloride dihydrate | 0.75 g/L |

pH was adjusted to 4.5 by adding ammonia gas during the culture. After the saccharide in the medium was consumed and depleted, a 700 g/L of glucose aqueous solution was continuously added (6 ml/hr).

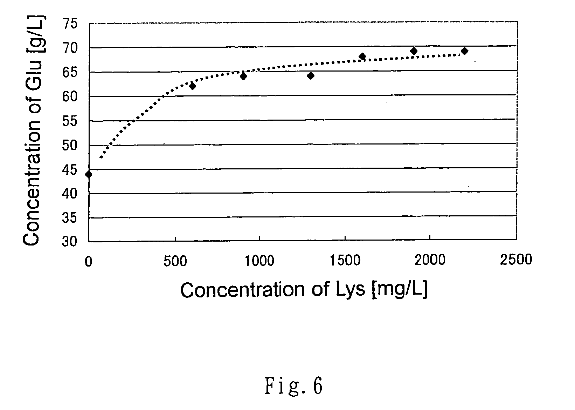

After the L-glutamic acid concentration in the medium exceeded 40 g/L, L-lysine hydrochloride was added in an amount of 0 to 0.9 g per 300 ml of the culture medium. Then, the L-glutamic acid concentration in the supernatant of the culture medium was measured with time. The supernatant of the culture medium was a supernatant liquid obtained by centrifuging the culture medium at 10000 x G for 1 minute. On the other hand, the polymorphism of crystals precipitated in the culture medium was determined by microscopic observation. The L-glutamic acid concentration in the culture medium increases as the culture time passes, and also the L-glutamic acid concentration in the supernatant liquid decreases as the crystals are precipitated. Therefore, the maximum concentration of L-glutamic acid in the supernatant was defined as a precipitation concentration thereof. The results are shown in Fig. 6.

In a culture system at pH 4.5, 44 g/L of β-form crystals of L-glutamic acid is precipitated in an L-lysine-free culture medium. The solubility of L-glutamic acid at pH 4.5 is 40 g/L. Therefore, in the case of adding seed crystals, the addition of seed crystals should be performed at concentrations ranging from 40 g/L to 44 g/L. This range corresponds to 50 minutes when the production rate of L-glutamic acid in a culture is 5g/L/h, so that extremely precise control will be required also in consideration of an analytical error. In contrast, in a system in the presence of 600 mg/L of L-lysine, the concentration at which precipitation of β-form crystals occurs increases to 62 g/L and the permissible range of seed-crystal addition largely extends to 40 g/L to 62 g/L. This corresponds to 4 hours when the production rate of L-glutamic acid is 5 g/L/h, so that the control will become extremely easy. Furthermore, the super solubility of L-glutamic acid increases as the concentration of L-lysine increases (Fig. 6).

The above phenomenon is considered to occur as a result of inhibiting crystal growth by adsorption of L-lysine on the crystal plane of a-form crystals of L-glutamic acid. Thus, a large super saturation is required to attain the crystal growth (including the generation of nucleus) enough to merge an increase in glutamic-acid concentration with culture. Therefore, it is presumed that the super saturation increases as the concentration of L-lysine increases.

When the concentration of L-lysine added to the medium exceeded 900 mg/L, the natural crystallization of a-form crystals could be found even without the addition of seed crystals. This is supposedly because L-lysine may inhibit crystal growth by its strong adsorption on the surface of β-form crystals rather than a-form crystals at pH 4.5 as its properties. Accordingly, it is presumed that the crystal growth of β-form crystals is suppressed to allow the a-form crystals to be crystallized and grown due to the presence of L-lysine at a certain concentration or more.

### (2) Control of crystal form of L-glutamic acid crystals with L-lysine

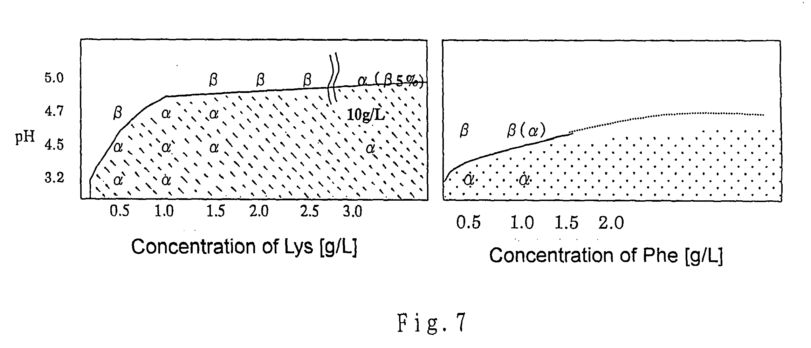

Next, L-glutamic acid was dissolved in water so as to obtain a super saturation (Css/Cs[-]) of 2, and then the pH and the L-lysine concentration added to a medium were changed to investigate the crystal form of L-glutamic acid crystals precipitated in a cooling-crystallization system (34°C). The adjustment of pH was performed by addition of ammonia water. In addition, instead of L-lysine, L-phenylalanine was added and the same experiment was performed. The results are shown in Fig. 7. Furthermore, the solubility and the glutamic-acid concentration at each pH are listed in Table 1.

As shown in Fig. 7, at pH 4.5, it was found that the crystal form precipitated by the addition of L-lysine could be controlled to a-form. On the other hand, L-phenylalanine showed the same effect at pH 3.2 but such an effect could not be observed at pH 4.5 or more. The difference between the two may be caused because the amino acids induce different dissociation states at each pH.

**Table 1**

| pH | Solubility (g/100g H₂O) | L-glutamic acid concentration(g/100g H₂O) |
|---|---|---|
| 3.2 | 1.2 | 2.4 |
| 4.5 | 4 | 8 |
| 4.7 | 6 | 12 |
| 5 | 10 | 20 |

### Industrial Applicability

In the method for producing L-glutamic acid by fermentation to produce and accumulate L-glutamic acid while precipitating L-glutamic acid in a medium, a-form crystals of L-glutamic acid can be precipitated without addition of seed crystals by making a certain amount or more of L-lysine exist in the medium.

Also, in the case of adding the seed crystals in the medium, it is easy to control the timing at which the seed crystals are added by making L-lysine exist in the medium.

## Claims

1. A method for producing L-glutamic acid by fermentation, which comprises culturing a microorganism that can metabolize a carbon source at a specific pH in a liquid medium containing L-glutamic acid at a saturation concentration and the carbon source and has an ability to accumulate L-glutamic acid in a liquid medium having said pH in an amount exceeding the amount corresponding to said saturation concentration of L-glutamic acid in a medium of which pH is controlled so that L-glutamic acid is precipitated to cause accumulation of L-glutamic acid in the medium while precipitating the L-glutamic acid,
wherein the method comprises making L-lysine exist in the medium when L-glutaminc acid concentration in the medium is lower than the concentration at which natural crystallization of L-glutamic acid occurs so that a-form crystals of L-glutamic acid is precipitated.

2. The method according to claim 1, wherein the microorganism belongs to the genus *Pantoea.*

3. The method according to claim 2, wherein the microorganism is *Pantoea ananatis.*

4. The method according to any one of claims 1 to 3, wherein the amount of added L-lysine is 900 mg/L or more.

5. The method according to any one of claims 1 to 4, wherein pH of the medium at the time of making L-lysine exist is 3.0 to 5.0.

6. The method according to any one of claims 1 to 5, wherein an operation of making the a-form crystals of the L-glutamic acid exist in the medium is performed before occurrence of natural crystallization of the L-glutamic acid.
